# EUROPEAN PATENT APPLICATION

(11) **EP 4 119 556 A1**
(43) Date of publication of application: **18.01.2023**
(21) Application number: 21768863.9
(22) Date of filing: 12.03.2021
(51) Int. Cl.: C07D 417/12, A61K 31/4439, A61P 35/00

(54) **PHARMACEUTICAL COMPOSITION FOR PREVENTING OR TREATING CANCER COMPRISING AZOLE DERIVATIVES OR PHARMACEUTICALLY ACCEPTABLE SALT THEREOF**

(30) Priority: 13.03.2020 KR 20200031630
(71) Applicant: Yungjin Pharm. Co., Ltd., Seoul 05510 (KR)
(72) Inventor: LEE, Kwang Ok, Yongin-si Gyeonggi-do 16937 (KR); YOO, Ja Kyung, Yongin-si Gyeonggi-do 17097 (KR); LEE, Jun Hee, Seoul 05036 (KR); LEE, Mijung, Hwaseong-si Gyeonggi-do 18452 (KR); LEE, Kangwoo, Osan-si Gyeonggi-do 18113 (KR); MIN, Ji Eun, Suwon-si Gyeonggi-do 16590 (KR)
(74) Representative: Heller, Benjamin Henry
(86) International application number: PCT/KR2021/003112
(87) International publication number: WO 2021/182918

(57) **Abstract**

The present invention relates to a pharmaceutical composition for preventing or treating cancer, wherein the pharmaceutical composition comprises a thiazole derivative or a pharmaceutically acceptable salt thereof. Specifically, the present invention relates to a pharmaceutical composition that selectively inhibits the activity of CDK7 to reduce side effects, and treats and prevents cancer by inhibiting the proliferation of all cancer cells, rather than being limited to specific cancer cells.

## Description

### Technical Field

The present invention relates to a pharmaceutical composition for preventing or treating cancer, wherein the pharmaceutical composition comprises a thiazole derivative or a pharmaceutically acceptable salt thereof.

### Background Art

Protein kinase refers to a protein that plays a central role in the regulation of a wide variety of cellular processes and control of cellular functions. The activation of kinase is observed in a number of disease states, including benign and malignant proliferative disorders, as well as those resulting from inappropriate activation of the immune and nervous systems. Tumor development is closely related to cyclin-dependent kinase (CDK), a serine/threonine protein kinase family that plays an important role in cell proliferation by promoting cell cycle progression, and genetic modification of its regulators, and the like. This suggests that a CDK inhibitor may be a useful anticancer therapeutic agent.

CDKs form a complex with cyclin proteins and activate them by phosphorylation of serine or threonine residues on the substrate. Among them, CDK1, CDK2, CDK4, and CDK6 regulate entry into each phase of the cell cycle by phosphorylation of molecules important in the cell cycle. For example, CDK4/cyclinD and CDK6/cyclinD act on the G1 phase, CDK2/cyclinE is essential to enter the S phase, and CDK2/cyclinA is essential just before the transition from S phase to G2 phase. In addition, CDK8 and CDK9 are involved in gene transcription. The activation of such CDK is completed by phosphorylation by CDK-activating kinase (CAK), and the CDK7/cyclinH complex acts as a CAK

In addition, CDK7 phosphorylates the C-terminal domain (CTD) of RNA polymerase (RNAP) II, thereby promoting the expression of key oncogenes such as Myc. The oncogene Myc is aberrantly activated in many cancer cells and upregulates the expression of oncogenic transcription factors that induce cancer cell growth and survival and the expression of genes encoding proteins such as PD-L1 or CD47 that enable immune evasion, thereby promoting the growth of cancer cells.

That is, CDK7 is a CDK that acts as a CAK and phosphorylates CTD at the same time to regulate the cell cycle and is involved in the transcription of genes related to the proliferation of cancer cells. The inhibition of CDK7 inhibits the progression of the cell cycle of cancer cells, inhibits CTD phosphorylation of RNAP II, and reduces the expression of oncogenes such as Myc, thereby inhibiting the expression of oncogenic transcription factors and the expression of immune checkpoint proteins responsible for immune evasion. Therefore, the compound that inhibits CDK7 can be developed as a drug for preventing and treating cancer by inhibiting the proliferation of all cancer cells, rather than being limited to specific cancer cells.

In addition, although cancer cells have increased genetic heterogeneity compared to normal cells, they can inhibit the growth of cancer cells or induce apoptosis by targeting driver mutations of major oncogenes. However, in the case of "transcriptional poisoning cancer cells" without specific driver mutations, the growth of cancer cells can be inhibited by transcriptional regulatory kinases such as CDK7 (Yubao Wang et al., Cell 163, 174-186, September 24, 2015). Therefore, there is a need for compounds that treat diseases and disorders associated with selective transcriptional CDKs, particularly CDK7.

Accordingly, the present inventors have completed the present invention by discovering that the thiazole compound of the present invention has a high CDK7 inhibitory activity and has an excellent effect in the treatment or prevention of cancer

### Prior Art Document

### Patent Document

(Patent Document 1) International Publication WO 2014/063068
(Patent Document 2) International Publication WO 2015/058140

### Detailed Description of Invention

### Technical Problem

An object of the present invention is to provide a pharmaceutical composition having an excellent effect in the prevention or treatment of cancer.

Specifically, another object of the present invention is to provide an excellent drug that can be widely utilized for the treatment and prevention of cancer by selectively inhibiting the activity of CDK7 to reduce side effects, and inhibiting the proliferation of all cancer cells, rather than being limited to specific cancer cells.

Therefore, another object of the present invention is to provide a pharmaceutical composition comprising a compound useful for the prevention or treatment of cancer by selectively inhibiting the activity of CDK7.

### Solution to Problem

The present invention provides a pharmaceutical composition for preventing or treating cancer, comprising a compound of Formula 1 below or a pharmaceutically acceptable salt thereof. 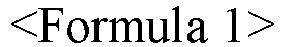 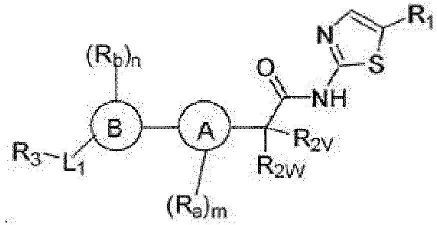 in the formula,
ring A is cycloalkyl, aryl, heteroaryl, or heterocycloalkyl;
ring B is cycloalkyl, aryl, heteroaryl, or heterocycloalkyl;
R₁ is H, halo, alkyl, cycloalkyl, heterocycloalkyl, aryl, heteroaryl, haloalkyl, cyano, - SR_{c}, -C(O)R_{c}, -C(O)OR_{c}, -C(O)N(R_{c})(R_{d}), -N(R_{c})(R_{d}), -(C(R_{g})₂)_{q}-N(R_{c})(R_{d}), -OR_{c}, or - (C(R_{g})₂)_{q}-OR_{c};
wherein R_{c} and R_{d} are each independently H, alkyl, cycloalkyl, aryl, heterocycloalkyl, or heteroaryl; or
R_{c} and R_{d} are taken together with the atoms to which they are attached to form an unsubstituted or substituted heterocycloalkyl;
q is an integer selected from 1 to 3; and
each R_{g} is independently H or alkyl;
R_{2V} and R_{2W} are each independently H, halo, hydroxy, alkyl, cycloalkyl, heterocycloalkyl, alkoxy, alkoxyalkyl, or amino; or
R_{2V} and R_{2W} are taken together with the atoms to which they are attached to form a cycloalkyl or heterocycloalkyl; and
L₁ is absent, -N(Rₑ)-, -^{∗}CH₂N(Rₑ)-, -^{∗}CH₂CH₂N(Rₑ)-, or alkylene, wherein ^{∗} is the position at which said L₁ is attached to ring B;
wherein Rₑ is H or alkyl; or Rₑ is bound to an atom of ring B to form a heteroaryl or heterocycloalkyl ring structure fused to ring B, which is unsubstituted or substituted;
R₃ is 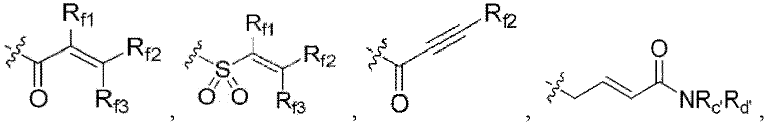 or 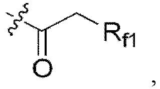
wherein R_{f1} is H, halo, alkyl, or cyano;
R_{f2} and R_{f3} are each independently H, halo, alkyl, cycloalkyl, heterocycloalkyl, heteroaryl, aryl, alkoxy, alkoxyalkyl, -N(R_{c'})(R_{d'}), or -(C(R_{g'})₂)_{q}-N(R_{c'})(R_{d'});
each R_{g'} is independently H or alkyl;
R_{c'} and R_{d'} are each independently H, alkyl, heterocycloalkyl, alkoxy, or alkoxyalkyl; or
R_{c'} and R_{d'} are taken together with the atoms to which they are attached to form an unsubstituted or substituted heterocycloalkyl,
each Rₐ is independently H, halo, hydroxyl, nitro, or cyano; or alkyl, alkoxy, alkoxyalkyl, amino, haloalkyl, aryl, heteroaryl, heterocycloalkyl, or cycloalkyl, which is unsubstituted or substituted; and
each R_{b} is independently H, halo, hydroxyl, nitro, or cyano; or alkyl, alkoxy, alkoxyalkyl, amino, haloalkyl, aryl, heteroaryl, heterocycloalkyl, cycloalkyl, or -NRₕRⱼ, which is unsubstituted or substituted;
wherein Rₕ and Rⱼ are each independently H, alkyl, -N(R_{h'})(R_{j'}), -(C(R_{g'})₂)_{q}-N(R_{h'})(R_{j'}), or heterocycloalkyl, and
R_{h'} and R_{j'} are each independently H or alkyl; or
R_{h'} and R_{j'} are taken together with the atoms to which they are attached to form an unsubstituted or substituted heterocycloalkyl; and
m and n are each independently an integer selected from 1 to 4.

### Effects of Invention

The compound of the present invention or a pharmaceutically acceptable salt thereof can inhibit the activity of CDK7, thereby inhibiting the phosphorylation of the C-terminal domain (CTD) of RNA polymerase (RNAP) II to inhibit the expression of oncogenes such as Myc and MCL1, as well as to inhibit the transcription of transcriptional poisoning cancer cells without specific driver mutations.

In addition, the compound of the present invention or a pharmaceutically acceptable salt thereof can inhibit the progression of the cancer cell cycle by inhibiting the activity of CDK7.

Therefore, the compound of the present invention or a pharmaceutically acceptable salt thereof selectively inhibits CDK7 to reduce side effects and exhibit excellent anticancer effects, and thus can be usefully utilized as a preventive or therapeutic agent for various cancers.

### Brief Description of Drawings

Fig. 1 illustrates the results obtained by confirming inhibition of the phosphorylation of the C-terminal domain of RNA polymerase (RNAP) II and the expression of oncogenes through Western blotting when TNBC (MDA-MB-468) cells were treated with the compound of the present invention.
Fig. 2 illustrates the results obtained by confirming inhibition of the phosphorylation of the C-terminal domain of RNA polymerase (RNAP) II and the expression of oncogenes through Western blotting when TNBC (MDA-MB-231) cells were treated with the compound of the present invention.
Fig. 3 illustrates the results obtained by confirming inhibition of the phosphorylation of the C-terminal domain of RNA polymerase (RNAP) II and the expression of oncogenes through Western blotting when HepG2 cells were treated with the compound of the present invention.
Fig. 4 illustrates the results obtained by confirming inhibition of the phosphorylation of the C-terminal domain of RNA polymerase (RNAP) II and the expression of oncogenes through Western blotting when MV-4-11 cells were treated with the compound of the present invention.
Fig. 5 illustrates the results obtained by confirming inhibition of the phosphorylation of the C-terminal domain of RNA polymerase (RNAP) II and the expression of oncogenes through Western blotting when MV-4-11 cells were treated with the compound of the present invention.
Fig. 6 illustrates the results obtained by confirming inhibition of the expression of cell cycle-related genes through Western blotting when TNBC (MDA-MB-231) cells were treated with the compound of the present invention.
Fig. 7 illustrates the results obtained by confirming the cell cycle-regulating efficacy through cell cycle measurement when TNBC (MDA-MB-231) cells were treated with the compound of the present invention.
Fig. 8 illustrates the occupancy rate of CDK7 depending on the compound exposure time when MV-4-11 cells were treated with the compound of the present invention.
Fig. 9 illustrates the activity of Caspase-3/7 depending on the compound exposure time when MV-4-11 cells were treated with the compound of the present invention.
Fig. 10 illustrates the cell growth inhibition rate depending on the compound exposure time when MV-4-11 cells were treated with the compound of the present invention.
Fig. 11 illustrates the survival rate when the compound of the present invention was administered to the MOLM-13 orthotopic transplantation model.
Fig. 12 illustrates the change in body weight when the compound of the present invention was administered to the MOLM-13 orthotopic transplantation model.

### Best Mode for Carrying out the Invention

Hereinafter, the present invention will be described in more detail.

Unless defined otherwise, all technical terms used herein have the same meaning as commonly understood by one of ordinary skill in the art to which the present invention belongs. Moreover, numerical values described herein are considered to include the meaning of "about" unless explicitly stated otherwise.

Definitions of moieties and substituents as used herein are provided below. Unless otherwise specified, each moiety and substituent has the following definitions and is used as commonly understood by one of ordinary skill in the art.

In accordance with convention used in the art, 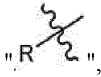 as used in the formulas herein, is used to indicate that a moiety or substituent "R" is attached to the backbone structure.

As used herein, the term "alkyl" is a hydrocarbon having unsubstituted or substituted primary, secondary, tertiary and/or quaternary carbon atoms, and includes a saturated aliphatic group which may be straight-chain, branched or cyclic, or a combination thereof. For example, an alkyl group may have from 1 to 20 carbon atoms (i.e., C₁-C₂₀ alkyl), from 1 to 10 carbon atoms (i.e., C₁-C₁₀ alkyl), or from 1 to 6 carbon atoms (i.e., C₁-C₆ alkyl), and preferably, an alkyl may be C₁-C₆ alkyl. Examples of suitable alkyl groups may include methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH3)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH3)CH₂CH₂CH₂CH₃), 3-hexyl (-CH(CH₂CH3)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃), and the like, but are not limited thereto. Moreover, as used throughout the specification, examples and claims, the term "alkyl" is intended to include both unsubstituted and substituted alkyl groups, the latter of which refers to an alkyl moiety having a substituent that replaces a hydrogen on at least one carbon of the hydrocarbon backbone, which includes a haloalkyl group such as trifluoromethyl, and the like.

As used herein, the term "C_{x-y}" or "Cₓ₋C_{y}," when used in conjunction with a chemical moiety such as acyl, acyloxy, alkyl, haloalkyl, cycloalkyl, alkenyl, alkynyl or alkoxy, is intended to include a group containing from x to y carbons in the chain. Co alkyl represents hydrogen when the substituent is in the terminal position or a bond when the substituent is in the internal position. In addition, for example, a C₁-C₆ alkyl group contains from 1 to 6 carbon atoms in the chain.

As used herein, the term "alkoxy" refers to a group in which an alkyl group is attached to the parent compound through an oxygen atom, which may be represented by -O-alkyl, wherein the alkyl group is as defined herein and may be unsubstituted or substituted. The alkyl group of an alkoxy group may have, for example, from 1 to 20 carbon atoms (i.e., C₁-C₂₀ alkoxy), from 1 to 12 carbon atoms (i.e., C₁-C₁₂ alkoxy), from 1 to 10 carbon atoms (i.e., C₁-C₁₀ alkoxy), or from 1 to 6 carbon atoms (i.e., C₁-C₆ alkoxy). Examples of suitable alkoxy groups may include methoxy (-O-CH₃ or -OMe), ethoxy (-OCH₂CH₃ or -OEt), and t-butoxy (-OC(CH₃)₃ or -O-tBu), and the like, but are not limited thereto.

As used herein, the term "alkoxyalkyl" refers to an alkyl group substituted with an alkoxy group, as defined herein, and may be represented by -alkyl-O-alkyl.

As used herein, the term "alkylene" refers to a saturated hydrocarbon group having a valence of 2, which may be branched, straight-chain, cyclic, or a combination thereof and is derived from the removal of two hydrogen atoms from the same carbon atom or two different carbon atoms of the parent alkane. For example, an alkylene group may have from 1 to 20 carbon atoms, from 1 to 10 carbon atoms, or from 1 to 6 carbon atoms. Examples of suitable alkylene groups may include methylene (-CH₂-) and 1,2-ethylene (-CH₂-CH₂-), and the like, but are not limited thereto.

As used herein, the term "amino" refers to -NH₂ in which a hydrogen atom is unsubstituted or substituted with a substituent such as alkyl, aryl, or the like, wherein the substituent such as alkyl, aryl, or the like, with which a hydrogen atom is substituted, is as defined herein and may be unsubstituted or substituted. Examples of suitable amino groups may include -NH₂, -N(CH₃)₂, -N(CH₃)-CH₂CH₂-N(CH₃)₂, and the like, but are not limited thereto.

As used herein, the term "aryl" refers to an unsubstituted or substituted, aromatic hydrocarbon group in which each atom of the ring is carbon and the ring is monocyclic, bicyclic or polycyclic. Preferably, an aryl ring may be a 6- to 14-membered ring or a 6- to 10-membered ring, and more preferably, it may be a 6-membered ring. An aryl group may be a polycyclic aryl having two or more rings in which two or more carbons are common to two adjacent rings. Examples of suitable aryl groups may include phenyl, naphtharenyl, phenanthryl, anthryl, and anilinyl, and the like, but are not limited thereto.

As used herein, the term "cycloalkyl" refers to an unsubstituted or substituted, non-aromatic saturated or unsaturated ring in which each atom of the ring is carbon and the ring is monocyclic, bicyclic or polycyclic. A cycloalkyl group may be a polycyclic cycloalkyl consisting of two or more rings in which one or more carbon atoms are common to adjacent rings. A polycyclic cycloalkyl may be a fused ring system, a spirocyclic ring system or a bridged ring system, wherein at least one of the rings is cycloalkyl and the other ring may be, for example, cycloalkyl, aryl, heteroaryl, and/or heterocycloalkyl, as defined herein. Examples of suitable cycloalkyl groups may include cyclopropyl, cyclobutyl, cyclopentyl, cyclohexyl, cycloheptyl, cyclooctyl, and the like, but are not limited thereto.

As used herein, the terms "halo" and "halogen" both refer to halogen and include chloro, fluoro, bromo, and iodo.

As used herein, the term "haloalkyl" is an alkyl group in which at least one of the hydrogen atoms of the alkyl group, as defined above, is replaced by a halogen atom. The alkyl moiety of a haloalkyl group may have from 1 to 20 carbon atoms (i.e., C₁-C₂₀ haloalkyl), from 1 to 12 carbon atoms (i.e., C₁-C₁₂ haloalkyl), from 1 to 10 carbon atoms (i.e., C₁-C₁₀ haloalkyl), or from 1 to 6 carbon atoms (i.e., C₁-C₆ haloalkyl). Examples of suitable haloalkyl groups may include -CF₃, -CHF₂, -CFH₂, and -CH₂CF₃, and the like, but are not limited thereto.

As used herein, the term "heteroaryl" refers to an unsubstituted or substituted aromatic group containing one or more heteroatoms in the ring, which is monocyclic, bicyclic or polycyclic. Non-limiting examples of suitable heteroatoms that may be contained in the aromatic ring may include N, O and S. A heteroaryl group may be a polycyclic heteroaryl consisting of two or more rings in which one or more atoms are common to adjacent rings. Examples of suitable heteroaryl groups may include benzofuranyl, benzothiophenyl, pyrrolo, furanyl, thiophenyl, imidazolyl, indolyl, isoindolyl, isoxazolyl, isothiazolyl, oxazolyl, thiazolyl, quinolinyl, isoquinolinyl, pyrazolyl, pyridinyl, pyrazinyl, pyridazinyl, and pyrimidinyl, and the like, but are not limited thereto.

As used herein, the term "heterocycloalkyl" refers to an unsubstituted or substituted, non-aromatic, saturated or partially saturated ring containing one or more heteroatoms in the ring, which is monocyclic, bicyclic or polycyclic. A heterocycloalkyl group may be a polycyclic heterocycloalkyl consisting of two or more rings in which one or more atoms are common to adjacent rings. A polycyclic heterocycloalkyl may be a fused ring system, a spirocyclic ring system or a bridged ring system, wherein at least one of the rings is heterocycloalkyl and the other ring may be, for example, cycloalkyl, aryl, heteroaryl, and/or heterocycloalkyl, as defined herein. Examples of suitable heterocycloalkyl groups may include piperidinyl, piperazinyl, pyrrolidinyl, morpholinyl, lactonyl, lactamyl, azetidinyl, dihydropyridinyl, dihydroindolyl, tetrahydropyridinyl (piperidinyl), tetrahydrothiophenyl, sulfur-oxidized tetrahydrothiophenyl, indolenyl, 4-piperidinyl, 2-pyrrolidonyl, tetrahydrofuranyl, tetrahydroquinolinyl, tetrahydroisoquinolinyl, decahydroquinolinyl, octahydroisoquinolinyl, 6H-1,2,5-thiadiazinyl, 2H,6H-1,5,2-dithiazinyl, pyranyl, chromenyl, xanthenyl, phenoxatinyl, 2H-pyrrolyl, 3H-indolyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, phenanthrolinyl, phenazinyl, phenothiazinyl, furazanyl, phenoxazinyl, isochromanyl, chromanyl, imidazolidinyl, pyrazolidinyl, pyrazolinyl, quinuclidinyl, and oxazolidinyl, and the like, but are not limited thereto.

As used herein, the term "substituted", for example "substituted alkyl," means that one or more hydrogen atoms of the alkyl are each independently replaced by a non-hydrogen substituent. A substituent may include any of the substituents described herein, for example, halogen, hydroxyl, alkyl, hydroxyalkyl, haloalkyl, cyanoalkyl, alkoxyalkyl, carbonyl (for example, carboxyl, alkoxycarbonyl, formyl, or acyl), thiocarbonyl (for example, thioester, thioacetate, or thioformate), alkoxyl, phosphoryl, phosphate, phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, aryl, or heteroaryl, but is not limited thereto. A substituted moiety on the hydrocarbon chain may optionally itself be substituted.

As used herein, the term "pharmaceutically acceptable salt" refers to any acid addition salt or base addition salt, which is nontoxic and harmless to the patient and the side effects attributable to said salt do not diminish the beneficial efficacy of the compound of the present invention. Inorganic acids that form suitable salts may include hydrochloric acid, phosphoric acid, sulfuric acid, nitric acid, tartaric acid, hydrobromic acid, hydroiodic acid, nitrous acid, phosphorous acid, and the like. Organic acids that form suitable salts may include glycolic acid, lactic acid, pyruvic acid, malonic acid, succinic acid, fumaric acid, malic acid, tartaric acid, citric acid, ascorbic acid, maleic acid, benzoic acid, phenylacetic acid, cinnamic acid, salicylic acid, nicotinic acid, tosylic acid, camphorsulfonic acid, naphthoic acid, acetic acid, trifluoroacetic acid, oxalic acid, mandelic acid, propionic acid, citric acid, lactic acid, gluconic acid, galacturonic acid, glutamic acid, glutaric acid, glucuronic acid, aspartic acid, carbonic acid, vanillic acid, benzenesulfonic acid, p-toluenesulfonic acid, or methanesulfonic acid, and the like. Preferably, it may be oxalate, hydrochloride, benzenesulfonate, hemifumarate, succinate, hemimaleate, nicotinate, tosylate, glycolate, hemisulfonate, tartrate, maleate, aspartate, malate, citrate, malonate, phosphate, glutamate, camphorsulfonate, 3-hydroxy-2-naphthoate, mesylate, or 4-hydroxy-benzoate, but is not limited thereto.

As used herein, the term "prevention" refers to any action of inhibiting or delaying the onset of a disease by administration of a compound or composition, and the like, and "treatment" refers to any action in which symptoms of a subject suspected of and suffering from a disease are improved or changed to a beneficial effect by administration of a compound or composition, and the like.

### Thiazole derivative compound

The present invention relates to a pharmaceutical composition for preventing or treating cancer, wherein the pharmaceutical composition comprises a thiazole derivative compound or a pharmaceutically acceptable salt thereof. The thiazole derivative compound of the present invention is represented by Formula 1 below. 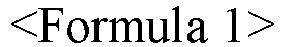 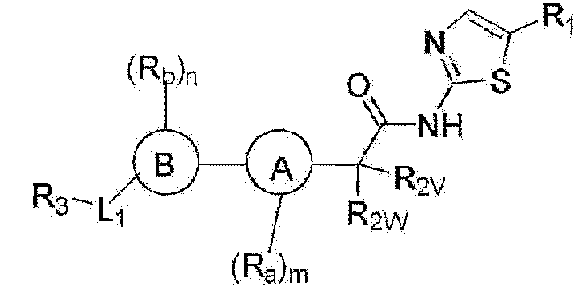 in the formula,
ring A is cycloalkyl, aryl, heteroaryl, or heterocycloalkyl;
ring B is cycloalkyl, aryl, heteroaryl, or heterocycloalkyl;
R₁ is H, halo, alkyl, cycloalkyl, heterocycloalkyl, aryl, heteroaryl, haloalkyl, cyano, - SR_{c}, -C(O)R_{c}, -C(O)OR_{c}, -C(O)N(R_{c})(R_{d}), -N(R_{c})(R_{d}), -(C(R_{g})₂)_{q}-N(R_{c})(R_{d}), -OR_{c}, or -
(C(R_{g})₂)_{q}-OR_{c};
wherein R_{c} and R_{d} are each independently H, alkyl, cycloalkyl, aryl, heterocycloalkyl, or heteroaryl; or
R_{c} and R_{d} are taken together with the atoms to which they are attached to form an unsubstituted or substituted heterocycloalkyl;
q is an integer selected from 1 to 3; and
each R_{g} is independently H or alkyl;
R_{2V} and R_{2W} are each independently H, halo, hydroxy, alkyl, cycloalkyl, heterocycloalkyl, alkoxy, alkoxyalkyl, or amino; or
R_{2V} and R_{2W} are taken together with the atoms to which they are attached to form a cycloalkyl or heterocycloalkyl; and
L₁ is absent, -N(Rₑ)-, -^{∗}CH₂N(Rₑ)-, -^{∗}CH₂CH₂N(Rₑ)-, or alkylene, wherein ^{∗} is the position at which said L₁ substituent is attached to ring B;
wherein Rₑ is H or alkyl; or Rₑ is bound to an atom of ring B to form a heteroaryl or heterocycloalkyl ring structure fused to ring B, which is unsubstituted or substituted;
R₃ is 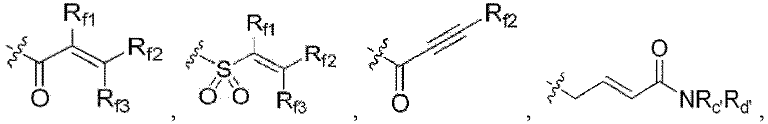 or 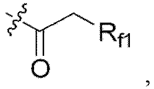
wherein R_{f1} is H, halo, alkyl, or cyano;
R_{f2} and R_{f3} are each independently H, halo, alkyl, cycloalkyl, heterocycloalkyl, heteroaryl, aryl, alkoxy, alkoxyalkyl, -N(R_{c'})(R_{d'}), or -(C(R_{g'})₂)_{q}-N(R_{c'})(R_{d'});
each R_{g'} is independently H or alkyl;
R_{c'} and R_{d'} are each independently H, alkyl, heterocycloalkyl, alkoxy, or alkoxyalkyl; or
R_{c'} and R_{d'} are taken together with the atoms to which they are attached to form an unsubstituted or substituted heterocycloalkyl,
each Rₐ is independently H, halo, hydroxyl, nitro, or cyano; or alkyl, alkoxy, alkoxyalkyl, amino, haloalkyl, aryl, heteroaryl, heterocycloalkyl, or cycloalkyl, which is unsubstituted or substituted; and
each R_{b} is independently H, halo, hydroxyl, nitro, or cyano; or alkyl, alkoxy, alkoxyalkyl, amino, haloalkyl, aryl, heteroaryl, heterocycloalkyl, cycloalkyl, or -NRₕRⱼ, which is unsubstituted or substituted;
wherein Rₕ and Rⱼ are each independently H, alkyl, -N(R_{h'})(R_{j'}), -(C(R_{g'})₂)_{q}-N(R_{h'})(R_{j'}), or heterocycloalkyl, and
R_{h'} and R_{j'} are each independently H or alkyl; or
R_{h'} and R_{j'} are taken together with the atoms to which they are attached to form an unsubstituted or substituted heterocycloalkyl; and
m and n are each independently an integer selected from 1 to 4.

In one embodiment, ring A may be 6- to 10-membered aryl; 5- or 6-membered heteroaryl containing 1 to 4 heteroatoms independently selected from the group consisting of N, O and S; or 5- or 6-membered heterocycloalkyl containing 1 to 4 heteroatoms independently selected from the group consisting of N, O and S, and preferably, ring A may be phenyl, pyridinyl, pyrazinyl, pyrazolyl, thiophenyl, thiazolyl, or piperidinyl.

In one embodiment, ring B may be 6- to 10-membered aryl; 5- or 6-membered heteroaryl containing 1 to 4 heteroatoms independently selected from the group consisting of N, O and S; or 5- or 6-membered heterocycloalkyl containing 1 to 4 heteroatoms independently selected from the group consisting of N, O and S, and preferably, ring B may be phenyl, pyridinyl, pyrimidinyl, pyrazinyl, pyridazinyl, pyrazolyl, imidazolyl, thiazolyl, tetrahydropyridinyl, piperidinyl, or piperazinyl.

In one embodiment, R₁ may be halo, C₁-C₆ alkyl, C₃-C₇ cycloalkyl, C₁-C₆ haloalkyl, cyano, -SR_{c}, -C(O)R_{c}, -C(O)OR_{c}, -N(R_{c})(R_{d}), -OR_{c}, or -C(R_{g})₂-OR_{c}, wherein R_{c} and R_{d} may be each independently H or C₁-C₆ alkyl, and each R_{g} may be independently H or C₁-C₆ alkyl.

In one embodiment, R_{2V} and R_{2W} may be each independently H, halo, or C₁-C₆ alkyl; or R_{2V} and R_{2W} may be taken together with the atoms to which they are attached to form C₃-C₇ cycloalkyl.

In one embodiment, L₁ may be absent, -N(Rₑ)-, or -^{∗}CH₂N(Rₑ)-, wherein ^{∗} may be the position at which said L₁ is attached to ring B; wherein Rₑ may be H or C₁-C₆ alkyl; or Rₑ may be bound to an atom of ring B to form, as a ring fused to ring B, 5- or 6-membered heteroaryl containing 1 to 4 heteroatoms independently selected from the group consisting of N, O and S; or 5- or 6-membered heterocycloalkyl containing 1 to 4 heteroatoms independently selected from the group consisting of N, O and S; wherein heteroaryl or heterocycloalkyl may be unsubstituted or substituted with C₁-C₆ alkyl.

In one embodiment, L₁ may be absent, -N(Rₑ)-, or -^{∗}CH₂N(Rₑ)-, wherein ^{∗} may be the position at which said L₁ is attached to ring B; wherein Rₑ may be H or C₁-C₆ alkyl; or Rₑ may be bound to an atom of ring B to form, as a ring fused to ring B, pyrrolidine or pyrrole, wherein pyrrolidine or pyrrole may be unsubstituted or substituted with C₁-C₆ alkyl.

In one embodiment, R₃ may be 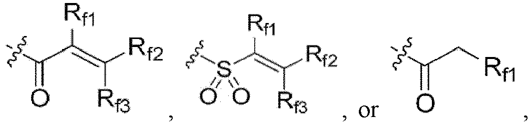 wherein R_{f1} may be H, C₁-C₆ alkyl, or cyano; and R_{f2} and R_{f3} may be each independently H, C₁-C₆ alkyl, C₃-C₇ cycloalkyl, -N(R_{c'})(R_{d'}), or -(CH₂)N(R_{c'})(R_{d'}); wherein R_{c'} and R_{d'} may be each independently H or C₁-C₆ alkyl; or R_{c'} and R_{d'} may be taken together with the atoms to which they are attached to form 4- to 7-membered heterocycloalkyl containing 1 to 3 heteroatoms independently selected from the group consisting of N, O and S, wherein heterocycloalkyl may be unsubstituted or substituted with halo, hydroxy, C₁-C₆ alkyl, or C₁-C₆ alkoxy-C₁-C₆ alkyl.

In one embodiment, R₃ may be 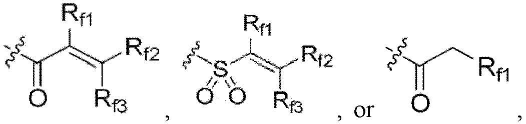 wherein R_{f1} may be H, C₁-C₆ alkyl, or cyano; and R_{f2} and R_{f3} may be each independently H, C₁-C₆ alkyl, C₃-C₇ cycloalkyl, -N(CH₃)₂, or -(CH₂)N(R_{c}')(R_{d}'); wherein R_{c'} and R_{d'} may be each independently C₁-C₆ alkyl; or R_{c'} and R_{d'} may be taken together with the atoms to which they are attached to form azetidinyl; piperidinyl; morpholinyl; piperazinyl unsubstituted or substituted with C₁-C₆ alkyl; 2,5-diazabicyclo[2.2.1]heptanyl unsubstituted or substituted with C₁-C₆ alkyl; 2-oxa-5-azabicyclo[2.2.1]heptanyl; or pyrrolidinyl unsubstituted or substituted with halo, hydroxy, or C₁-C₆ alkoxy-C₁-C₆ alkyl.

In one embodiment, each Rₐ may be independently H, halo, or C₁-C₆ alkyl; and each R_{b} may be independently H, halo, C₁-C₆ alkyl, C₃-C₇ cycloalkyl, C₁-C₆ alkoxy, cyano, C₁-C₆ haloalkyl, -NRₕRⱼ, or 5- to 6-membered heterocycloalkyl containing 1 to 4 heteroatoms independently selected from the group consisting of N, O and S, wherein heterocycloalkyl may be unsubstituted or substituted with C₁-C₆ alkyl, wherein Rₕ and Rⱼ may be each independently C₁-C₆ alkyl or -CH₂CH₂N(R_{h'})(R_{j'}); and R_{h'} and R_{j'} may be each independently H or C₁-C₆ alkyl.

In one embodiment, each Rₐ may be independently H, halo, or C₁-C₆ alkyl; and each R_{b} may be independently H, halo, C₁-C₆ alkyl, C₃-C₇ cycloalkyl, C₁-C₆ alkoxy, cyano, C₁-C₆ haloalkyl, -N(CH₃)-CH₂CH₂-N(CH₃)₂, or piperazinyl unsubstituted or substituted with C₁-C₆ alkyl.

In one embodiment, m and n may be 1.

In one embodiment, the compound of Formula 1 may be the compounds of Nos. 1 to 149 below.
1) N-(5-(3-(1-((5-methylthiazol-2-yl)amino)-1-oxopropan-2-yl)phenyl)pyridin-2-yl)acrylamide;
2) N-(5-(3-(1-((5-cyclopropylthiazol-2-yl)amino)-1-oxopropan-2-yl)phenyl)pyridin-2-yl)acrylamide;
3) (R)-N-(5-(3-(1-((5-cyclopropylthiazol-2-yl)amino)-1-oxopropan-2-yl)phenyl)pyridin-2-yl)acrylamide;
4) N-(5-(3-(1-((5-ethylthiazol-2-yl)amino)-1-oxopropan-2-yl)phenyl)pyridin-2-yl)acrylamide;
5) (E)-4-(dimethylamino)-N-(5-(3-(1-((5-methylthiazol-2-yl)amino)-1-oxopropan-2-yl)phenyl)pyridin-2-yl)but-2-enamide;
6) (E)-N-(5-(3-(1-((5-cyclopropylthiazol-2-yl)amino)-1-oxopropan-2-yl)phenyl)pyridin-2-yl)-4-(dimethylamino)but-2-enamide;
7) (E)-4-(dimethylamino)-N-(5-(3-(1-((5-methylthiazol-2-yl)amino-1-oxopropan-2-yl)phenyl)pyridin-2-yl)but-2-enamide hydrochloride;
8) N-(3-fluoro-3'-(1-((5-methylthiazol-2-yl)amino-1-oxopropan-2-yl)-[1,1'-biphenyl]-4-yl)acrylamide;
9) 2-(3-(1-acryloylindolin-5-yl)phenyl)-N-(5-methylthiazol-2-yl)propanamide;
10) (E)-N-(5-(3-(1,1-difluoro-2-((5-methylthiazol-2-yl)amino)-2-oxoethyl)phenyl)pyridin-2-yl)-4-(dimethylamino)but-2-enamide;
11) N-(6-(3-(1-((5-cyanothiazol-2-yl)amino)-1-oxopropan-2-yl)-4-fluorophenyl)pyridazin-3-yl)acrylamide;
12) 2-(3-(6-acrylamidopyridazin-3-yl)phenyl)-N-(5-cyanothiazol-2-yl)-3-methylbutanamide;
13) N-(6-(5-(1-((5-cyanothiazol-2-yl)amino)-1-oxopropan-2-yl)-2-fluorophenyl)pyridazin-3-yl)acrylamide;
14) (S)-N-(5-(3-(1-(5-cyclopropylthiazol-2-yl)amino)-1-oxopropan-2-yl)phenyl)pyridin-2-yl)acrylamide;
15) (S,E)-N-(5-(3-(1-(5-cyclopropylthiazol-2-yl)amino)-1-oxopropan-2-yl)phenyl)pyridin-2-yl)-4-(dimethylamino)but-2-enamide;
16) (S)-N-(6-(3-(1-(5-cyclopropylthiazol-2-yl)amino)-1-oxopropan-2-yl)phenyl)pyrazin-2-yl)acrylamide;
17) (S,E)-4-(dimethylamino)-N-(5-(3-(1-((5-ethylthiazol-2-yl)amino)-1-oxopropan-2-yl)phenyl)pyridin-2-yl)but-2-enamide;
18) (S)-N-(5-(3-(1-((5-cyanothiazol-2-yl)amino)-1-oxopropan-2-yl)phenyl)pyridin-2-yl)acrylamide;
19) (S)-2-(3-(6-acrylamidopyridin-3-yl)phenyl)-N-(5-ethylthiazol-2-yl)butanamide;
20) (S)-2-(4'-acrylamido-3'-fluoro-[1,1'-biphenyl]-3-yl)-N-(5-ethylthiazol-2-yl)butanamide;
21) (S,E)-N-(5-(3-(1-((5-cyanothiazol-2-yl)amino)-1-oxopropan-2-yl)phenyl)pyridin-2-yl)-4-(dimethylamino)but-2-enamide;
22) (S)-N-(5-(3-(1-(5-cyclopropylthiazol-2-yl)amino)-1-oxopropan-2-yl)phenyl)pyrazin-2-yl)acrylamide;
23) (S)-N-(3'-(1-((5-cyclopropylthiazol-2-yl)amino)-1-oxopropan-2-yl)-[1,1'-biphenyl]-4-yl)acrylamide;
24) (S)-N-(3'-(1-((5-cyclopropylthiazol-2-yl)amino)-1-oxopropan-2-yl)-3-fluoro-[1,1'-biphenyl]-4-yl)acrylamide;
25) (S)-N-(5-(3-(1-((5-cyanothiazol-2-yl)amino)-1-oxopropan-2-yl)phenyl)pyrazin-2-yl)acrylamide;
26) (S)-N-(6-(3-(1-((5-cyanothiazol-2-yl)amino)-1-oxopropan-2-yl)phenyl)pyrazin-2-yl)acrylamide;
27) (S)-N-(5-(3-(1-(5-cyclopropylthiazol-2-yl)amino)-1-oxopropan-2-yl)phenyl)pyrimidin-2-yl)acrylamide;
28) (S)-N-(6-(3-(1-(5-cyclopropylthiazol-2-yl)amino)-1-oxopropan-2-yl)phenyl)pyridazin-3-yl)acrylamide;
29) (S)-N-(5-(3-(1-(5-cyclopropylthiazol-2-yl)amino)-1-oxopropan-2-yl)phenyl)-3-fluoropyridin-2-yl)acrylamide;
30) (S)-N-(3-cyano-3'-(1-((5-ethylthiazol-2-yl)amino)-1-oxopropan-2-yl)-[1,1'-biphenyl]-4-yl)acrylamide;
31) (S)-N-(5-(3-(1-((5-ethylthiazol-2-yl)amino)-1-oxopropan-2-yl)phenyl)-6-fluoropyridin-2-yl)acrylamide;
32) (S)-N-(6-(3-(1-((5-cyanothiazol-2-yl)amino)-1-oxopropan-2-yl)phenyl)pyridazin-3-yl)acrylamide;
33) (S)-N-(5-(3-(1-((5-cyanothiazol-2-yl)amino)-1-oxopropan-2-yl)phenyl)-3-fluoropyridin-2-yl)acrylamide;
34) (S)-N-(5-(3-(1-((5-cyanothiazol-2-yl)amino)-1-oxopropan-2-yl)phenyl)-3-methylpyrazin-2-yl)acrylamide;
35) (S)-N-(5-(3-(1-((5-isopropylthiazol-2-yl)amino)-1-oxopropan-2-yl)phenyl)pyridin-2-yl)acrylamide;
36) (S)-N-(5-(3-(1-((5-isopropylthiazol-2-yl)amino)-1-oxopropan-2-yl)phenyl)pyrazin-2-yl)acrylamide;
37) (S)-N-(5-(3-(1-oxo-1-((5-(trifluoromethyl)thiazol-2-yl)amino)propan-2-yl)phenyl)pyrazin-2-yl)acrylamide;
38) (S)-N-(5-(3-(1-oxo-1-((5-(trifluoromethyl)thiazol-2-yl)amino)propan-2-yl)phenyl)pyridin-2-yl)acrylamide;
39) (S)-N-(6-(3-(1-oxo-1-((5-(trifluoromethyl)thiazol-2-yl)amino)propan-2-yl)phenyl)pyridazin-3-yl)acrylamide;
40) (S)-N-(6-(3-(1-((5-isopropylthiazol-2-yl)amino)-1-oxopropan-2-yl)phenyl)pyridazin-3-yl)acrylamide;
41) N-(5'-(1-((5-cyanothiazol-2-yl)amino)-1-oxopropan-2-yl)-[3,3'-bipyridin]-6-yl)acrylamide;
42) N-(4-(1-((5-cyanothiazol-2-yl)amino)-1-oxopropan-2-yl)-[2,3'-bipyridin]-6'-yl)acrylamide;
43) N-(5-(5-(1-((5-cyclopropylthiazol-2-yl)amino)-1-oxopropan-2-yl)pyridin-3-yl)pyrazin-2-yl)acrylamide;
44) N-(5-(5-(1-((5-cyanothiazol-2-yl)amino)-1-oxopropan-2-yl)pyridin-3-yl)pyrazin-2-yl)acrylamide;
45) N-(5-(5-(1-oxo-1-((5-(trifluoromethyl)thiazol-2-yl)amino)propan-2-yl)pyridin-3-yl)pyrazin-2-yl)acrylamide;
46) N-(5-(5-(1-((5-ethylthiazol-2-yl)amino)-1-oxopropan-2-yl)pyridin-3-yl)pyrazin-2-yl)acrylamide;
47) N-(5-(5-(1-((5-isopropylthiazol-2-yl)amino)-1-oxopropan-2-yl)pyridin-3-yl)pyrazin-2-yl)acrylamide;
48) N-(5-(5-(1-((5-acetylthiazol-2-yl)amino)-1-oxopropan-2-yl)pyridin-3-yl)pyrazin-2-yl)acrylamide;
49) N-(6-(5-(1-oxo-1-((5-(trifluoromethyl)thiazol-2-yl)amino)propan-2-yl)pyridin-3-yl)pyridazin-3-yl)acrylamide;
50) N-(4-(1-((5-cyclopropylthiazol-2-yl)amino)-1-oxopropan-2-yl)-[2,3'-bipyridin]-6'-yl)acrylamide;
51) (R)-N-(5-(5-(1-oxo-1-((5-(trifluoromethyl)thiazol-2-yl)amino)propan-2-yl)pyridin-3-yl)pyrazin-2-yl)acrylamide;
52) (S)-N-(5-(5-(1-oxo-1-((5-(trifluoromethyl)thiazol-2-yl)amino)propan-2-yl)pyridin-3-yl)pyrazin-2-yl)acrylamide;
53) N-(5-(5-(1-oxo-1-((5-(trifluoromethyl)thiazol-2-yl)amino)propan-2-yl)thiophen-3-yl)pyridin-2-yl)acrylamide;
54) N-(5-(3-(1-((5-cyclopropylthiazol-2-yl)amino)-1-oxopropan-2-yl)-5-fluorophenyl)pyridin-2-yl)acrylamide;
55) N-(5-(5-(2-methyl-1-oxo-1-((5-(trifluoromethyl)thiazol-2-yl)amino)propan-2-yl)pyridin-3-yl)pyrazin-2-yl)acrylamide;
56) (S)-2-(3-(5-(2-cyanoacetamido)pyrazin-2-yl)phenyl)-N-(5-(trifluoromethyl)thiazol-2-yl)propanamide;
57) N-(5-(5-(1-oxo-1-((5-(trifluoromethyl)thiazol-2-yl)amino)propan-2-yl)thiophen-3-yl)pyrazin-2-yl)acrylamide;
58) (S)-2-(3-(5-(2-cyanoacetamido)pyrazin-2-yl)phenyl)-N-(5-cyanothiazol-2-yl)propanamide;
59) N-(5-(3-methyl-1-(1-oxo-1-((5-(trifluoromethyl)thiazol-2-yl)amino)propan-2-yl)-1H-pyrazol-4-yl)pyridin-2-yl)acrylamide;
60) 2-(1'-acryloyl-1',2',3',6'-tetrahydro-[3,4'-bipyridin]-5-yl)-N-(5-cyanothiazol-2-yl)propanamide;
61) N-(5-(5-(1-((5-chlorothiazol-2-yl)amino)-1-oxopropan-2-yl)pyridin-3-yl)pyrazin-2-yl)acrylamide;
62) (S)-N-(6-(3-(1-((5-cyclopropylthiazol-2-yl)amino)-1-oxopropan-2-yl)phenyl)pyridin-3-yl)acrylamide;
63) (E)-4-(dimethylamino)-N-(5-(5-(1-oxo-1-((5-(trifluoromethyl)thiazol-2-yl)amino)propan-2-yl)pyridin-3-yl)pyrazin-2-yl)but-2-enamide;
64) 2-(5-(5-(2-cyanoacetamido)pyrazin-2-yl)pyridin-3-yl)-N-(5-(trifluoromethyl)thiazol-2-yl)propanamide;
65) (E)-2-cyano-3-cyclopropyl-N-(5-(5-(1-oxo-1-((5-(trifluoromethyl)thiazol-2-yl)amino)propan-2-yl)pyridin-3-yl)pyrazin-2-yl)acrylamide;
66) N-(5-(5-(1-((5-methoxythiazol-2-yl)amino)-1-oxopropan-2-yl)pyridin-3-yl)pyrazin-2-yl)acrylamide;
67) N-(5-(5-(1-((5-fluorothiazol-2-yl)amino)-1-oxopropan-2-yl)pyridin-3-yl)pyrazin-2-yl)acrylamide;
68) N-(2-(4-methylpiperazin-1-yl)-4-(5-(1-oxo-1-((5-(trifluoromethyl)thiazol-2-yl)amino)propan-2-yl)pyridin-3-yl)phenyl)acrylamide;
69) (E)-2-cyano-3-(dimethylamino)-N-(5-(5-(1-oxo-1-((5-(trifluoromethyl)thiazol-2-yl)amino)propan-2-yl)pyridin-3-yl)pyrazin-2-yl)acrylamide;
70) N-(1-(5-(1-oxo-1-((5-(trifluoromethyl)thiazol-2-yl)amino)propan-2-yl)pyridin-3-yl)piperidin-4-yl)acrylamide;
71) N-(5-(5-(1-((5-(methylthio)thiazol-2-yl)amino)-1-oxopropan-2-yl)pyridin-3-yl)pyrazin-2-yl)acrylamide;
72) ethyl 2-(2-(5-(5-acrylamidopyrazin-2-yl)pyridin-3-yl)propanamido)thiazole-5-carboxylate;
73) (E)-N-(5-(5-(1-((5-chlorothiazol-2-yl)amino)-1-oxopropan-2-yl)pyridin-3-yl)pyrazin-2-yl)-4-(dimethylamino)but-2-enamide;
74) (E)-4-morpholino-N-(5-(5-(1-oxo-1-((5-(trifluoromethyl)thiazol-2-yl)amino)propan-2-yl)pyridin-3-yl)pyrazin-2-yl)but-2-enamide;
75) (E)-N-(5-(5-(1-oxo-1-((5-(trifluoromethyl)thiazol-2-yl)amino)propan-2-yl)pyridin-3-yl)pyrazin-2-yl)but-2-enamide;
76) N-(5-(5-(1-((5-chlorothiazol-2-yl)amino)-1-oxopropan-2-yl)thiophen-3-yl)pyridin-2-yl)acrylamide;
77) N-(5-(5-(1-((5-ethylthiazol-2-yl)amino)-1-oxopropan-2-yl)thiophen-3-yl)pyridin-2-yl)acrylamide;
78) N-(5-(5-(1-((5-acetylthiazol-2-yl)amino)-1-oxopropan-2-yl)thiophen-3-yl)pyridin-2-yl)acrylamide;
79) 2-(5-(5-propionamidopyrazin-2-yl)pyridin-3-yl)-N-(5-(trifluoromethyl)thiazol-2-yl)propanamide;
80) (E)-4-(5-methyl-2,5-diazabicyclo[2.2.1]heptan-2-yl)-N-(5-(5-(1-oxo-1-((5-(trifluoromethyl)thiazol-2-yl)amino)propan-2-yl)pyridin-3-yl)pyrazin-2-yl)but-2-enamide;
81) (S,E)-4-(dimethylamino)-N-(5-(3-(1-oxo-1-((5-(trifluoromethyl)thiazol-2-yl)amino)propan-2-yl)phenyl)pyrazin-2-yl)but-2-enamide;
82) N-(5'-(1-oxo-1-((5-(trifluoromethyl)thiazol-2-yl)amino)propan-2-yl)-[3,3'-bipyridin]-6-yl)acrylamide;
83) (E)-4-(2-oxa-5-azabicyclo[2.2.1]heptan-5-yl)-N-(5-(5-(1-oxo-1-((5-(trifluoromethyl)thiazol-2-yl)amino)propan-2-yl)pyridin-3-yl)pyrazin-2-yl)but-2-enamide;
84) N-(4-fluoro-5'-(1-oxo-1-((5-(trifluoromethyl)thiazol-2-yl)amino)propan-2-yl)-[3,3'-bipyridin]-6-yl)acrylamide;
85) 2-(5-(4-acryloylpiperazin-1-yl)pyridin-3-yl)-N-(5-(trifluoromethyl)thiazol-2-yl)propanamide;
86) 2-(5-(4-acryloylpiperazin-1-yl)pyridin-3-yl)-N-(5-ethylthiazol-2-yl)propanamide;
87) N-(6-(5-(1-((5-ethylthiazol-2-yl)amino)-1-oxopropan-2-yl)thiophen-3-yl)pyridin-3-yl)acrylamide;
88) N-(5-cyano-5'-(1-oxo-1-((5-(trifluoromethyl)thiazol-2-yl)amino)propan-2-yl)-[3,3'-bipyridin]-6-yl)acrylamide;
89) N-((5'-(1-oxo-1-((5-(trifluoromethyl)thiazol-2-yl)amino)propan-2-yl)-[3,3'-bipyridin]-6-yl)methyl)acrylamide;
90) N-(5'-(1-oxo-1-((5-(trifluoromethyl)thiazol-2-yl)amino)propan-2-yl)-[2,3'-bipyridin]-5-yl)acrylamide;
91) (S,E)-4-(dimethylamino)-N-(5-(3-(1-oxo-1-((5-(trifluoromethyl)thiazol-2-yl)amino)propan-2-yl)phenyl)pyrazin-2-yl)but-2-enamide;
92) (S,E)-4-morpholino-N-(5-(3-(1-oxo-1-((5-(trifluoromethyl)thiazol-2-yl)amino)propan-2-yl)phenyl)pyrazin-2-yl)but-2-enamide;
93) (S,E)-N-(5-(3-(1-((5-chlorothiazol-2-yl)amino)-1-oxopropan-2-yl)phenyl)pyrazin-2-yl)-4-(dimethylamino)but-2-enamide;
94) (E)-4-(2-oxa-5-azabicyclo[2.2.1]heptan-5-yl)-N-(5-(3-((S)-1-((5-chlorothiazol-2-yl)amino)-1-oxopropan-2-yl)phenyl)pyrazin-2-yl)but-2-enamide;
95) (S,E)-4-(4-methylpiperazin-1-yl)-N-(5-(3-(1-oxo-1-((5-(trifluoromethyl)thiazol-2-yl)amino)propan-2-yl)phenyl)pyrazin-2-yl)but-2-enamide;
96) (E)-4-(2-oxa-5-azabicyclo[2.2.1]heptan-5-yl)-N-(5-(3-((S)-1-oxo-1-((5-(trifluoromethyl)thiazol-2-yl)amino)propan-2-yl)phenyl)pyrazin-2-yl)but-2-enamide;
97) (S,E)-N-(5-(3-(1-((5-chlorothiazol-2-yl)amino)-1-oxopropan-2-yl)phenyl)pyrazin-2-yl)-4-(4-methylpiperazin-1-yl)but-2-enamide;
98) (S,E)-N-(5-(3-(1-((5-chlorothiazol-2-yl)amino)-1-oxopropan-2-yl)phenyl)pyrazin-2-yl)-4-morpholinobut-2-enamide;
99) (S,E)-4-(dimethylamino)-N-(3-fluoro-5-(3-(1-oxo-1-((5-(trifluoromethyl)thiazol-2-yl)amino)propan-2-yl)phenyl)pyridin-2-yl)but-2-enamide;
100) (S,E)-4-(dimethylamino)-N-(3-methoxy-5-(3-(1-oxo-1-((5-(trifluoromethyl)thiazol-2-yl)amino)propan-2-yl)phenyl)pyridin-2-yl)but-2-enamide;
101) (S,E)-N-(3-cyano-5-(3-(1-oxo-1-((5-(trifluoromethyl)thiazol-2-yl)amino)propan-2-yl)phenyl)pyridin-2-yl)-4-(dimethylamino)but-2-enamide;
102) (S,E)-N-(5-(3-(1-oxo-1-((5-(trifluoromethyl)thiazol-2-yl)amino)propan-2-yl)phenyl)pyrazin-2-yl)-4-(pyrrolidin-1-yl)but-2-enamide;
103) (S,E)-4-(azetidin-1-yl)-N-(5-(3-(1-oxo-1-((5-(trifluoromethyl)thiazol-2-yl)amino)propan-2-yl)phenyl)pyrazin-2-yl)but-2-enamide;
104) (S,E)-N-(5-(3-(1-((5-cyclobutylthiazol-2-yl)amino)-1-oxopropan-2-yl)phenyl)pyrazin-2-yl)-4-(dimethylamino)but-2-enamide;
105) (S,E)-4-(dimethylamino)-N-(5-(3-(1-((5-(2-hydroxypropan-2-yl)thiazol-2-yl)amino)-1-oxopropan-2-yl)phenyl)pyrazin-2-yl)but-2-enamide;
106) (S,E)-4-(dimethylamino)-N-(5-(3-(1-((5-(dimethylamino)thiazol-2-yl)amino)-1-oxopropan-2-yl)phenyl)pyrazin-2-yl)but-2-enamide;
107) (E)-4-(dimethylamino)-N-(5'-(1-oxo-1-((5-(trifluoromethyl)thiazol-2-yl)amino)propan-2-yl)-[3,3'-bipyridin]-6-yl)but-2-enamide;
108) (E)-N-(5-cyano-5'-(1-oxo-1-((5-(trifluoromethyl)thiazol-2-yl)amino)propan-2-yl)-[3,3'-bipyridin]-6-yl)-4-(dimethylamino)but-2-enamide;
109) (S,E)-4-(dimethylamino)-N-(3-methoxy-5-(3-(1-oxo-1-((5-(trifluoromethyl)thiazol-2-yl)amino)propan-2-yl)phenyl)pyrazin-2-yl)but-2-enamide;
110) (S,E)-2-(3-(1-(4-(dimethylamino)but-2-enoyl)-2,3-dihydro-1H-pyrrolo[2,3-b]pyridin-5-yl)phenyl)-N-(5-(trifluoromethyl)thiazol-2-yl)propanamide;
111) (S,E)-4-(dimethylamino)-N-(5-(3-(1-oxo-1-((5-(trifluoromethyl)thiazol-2-yl)amino)propan-2-yl)phenyl)-3-(trifluoromethyl)pyridin-2-yl)but-2-enamide;
112) (S,E)-4-(dimethylamino)-N-(3-methyl-5-(3-(1-oxo-1-((5-(trifluoromethyl)thiazol-2-yl)amino)propan-2-yl)phenyl)pyridin-2-yl)but-2-enamide;
113) (S,E)-N-(3-chloro-5-(3-(1-oxo-1-((5-(trifluoromethyl)thiazol-2-yl)amino)propan-2-yl)phenyl)pyridin-2-yl)-4-(dimethylamino)but-2-enamide;
114) (S,E)-4-(diethylamino)-N-(5-(3-(1-oxo-1-((5-(trifluoromethyl)thiazol-2-yl)amino)propan-2-yl)phenyl)pyrazin-2-yl)but-2-enamide;
115) (E)-4-((R)-3-fluoropyrrolidin-1-yl)-N-(5-(3-((S)-1-oxo-1-((5-(trifluoromethyl)thiazol-2-yl)amino)propan-2-yl)phenyl)pyrazin-2-yl)but-2-enamide;
116) (S,E)-4-(dimethylamino)-N-(5-(3-(1-oxo-1-((5-(trifluoromethyl)thiazol-2-yl)amino)propan-2-yl)phenyl)pyridin-2-yl)but-2-enamide;
117) (E)-4-((S)-3-fluoropyrrolidin-1-yl)-N-(5-(3-((S)-1-oxo-1-((5-(trifluoromethyl)thiazol-2-yl)amino)propan-2-yl)phenyl)pyrazin-2-yl)but-2-enamide;
118) (E)-4-((S)-2-(methoxymethyl)pyrrolidin-1-yl)-N-(5-(3-((S)-1-oxo-1-((5-(trifluoromethyl)thiazol-2-yl)amino)propan-2-yl)phenyl)pyrazin-2-yl)but-2-enamide;
119) (E)-4-(3-hydroxypyrrolidin-1-yl)-N-(5-(3-((S)-1-oxo-1-((5-(trifluoromethyl)thiazol-2-yl)amino)propan-2-yl)phenyl)pyrazin-2-yl)but-2-enamide;
120) (S,E)-N-(5-(3-(1-oxo-1-((5-(trifluoromethyl)thiazol-2-yl)amino)propan-2-yl)phenyl)pyrazin-2-yl)-4-(piperidin-1-yl)but-2-enamide;
121) (S,E)-N-(3-cyano-5-(3-(1-((5-ethylthiazol-2-yl)amino)-1-oxopropan-2-yl)phenyl)pyridin-2-yl)-4-(dimethylamino)but-2-enamide;
122) (S,E)-2-(3-(1-(4-(dimethylamino)but-2-enoyl)-2,3-dihydro-1H-pyrrolo[2,3-b]pyridin-5-yl)phenyl)-N-(5-ethylthiazol-2-yl)propanamide;
123) (E)-4-(2-oxa-5-azabicyclo[2.2.1]heptan-5-yl)-N-(3-cyano-5-(3-((S)-1-((5-ethylthiazol-2-yl)amino)-1-oxopropan-2-yl)phenyl)pyridin-2-yl)but-2-enamide;
124) (S)-N-(3-cyano-5-(3-(1-oxo-1-((5-(trifluoromethyl)thiazol-2-yl)amino)propan-2-yl)phenyl)pyridin-2-yl)acrylamide;
125) (S,E)-4-(dimethylamino)-N-(5-(3-(1-((5-ethylthiazol-2-yl)amino)-1-oxopropan-2-yl)phenyl)-3-methylpyridin-2-yl)but-2-enamide;
126) (S,E)-2-(3-(1-(4-(dimethylamino)but-2-enoyl)-2-methyl-1H-pyrrolo[2,3-b]pyridin-5-yl)phenyl)-N-(5-ethylthiazol-2-yl)propanamide;
127) (S,E)-2-(3-(1-(4-(dimethylamino)but-2-enoyl)-1H-pyrrolo[2,3-b]pyridin-5-yl)phenyl)-N-(5-ethylthiazol-2-yl)propanamide;
128) (E)-1-(3-(5-cyano-6-(4-(dimethylamino)but-2-enamido)pyridin-3-yl)phenyl)-N-(5-ethylthiazol-2-yl)cyclopropane-1-carboxamide;
129) (S,E)-4-(dimethylamino)-N-(5-(3-(1-((5-ethylthiazol-2-yl)amino)-1-oxopropan-2-yl)phenyl)pyridin-2-yl)-N-methylbut-2-enamide;
130) (S,E)-4-(dimethylamino)-N-(5-(3-(1-((5-ethylthiazol-2-yl)amino)-1-oxopropan-2-yl)phenyl)-3-fluoropyridin-2-yl)but-2-enamide;
131) (E)-4-(dimethylamino)-N-(6-(3-(1-((5-ethylthiazol-2-yl)amino)-1-oxobutan-2-yl)phenyl)pyridin-3-yl)but-2-enamide;
132) (S,E)-N-(3-cyano-5-(3-(1-((5-ethylthiazol-2-yl)amino)-1-oxopropan-2-yl)phenyl)pyridin-2-yl)-4-(dimethylamino)-N-methylbut-2-enamide;
133) (E)-1-(3-(1-(4-(dimethylamino)but-2-enoyl)-2,3-dihydro-1H-pyrrolo[2,3-b]pyridin-5-yl)phenyl)-N-(5-ethylthiazol-2-yl)cyclopropane-1-carboxamide;
134) (E)-1-(3-(6-(4-(dimethylamino)but-2-enamido)-5-fluoropyridin-3-yl)phenyl)-N-(5-ethylthiazol-2-yl)cyclopropane-1-carboxamide;
135) (S,E)-N-(3-cyano-5-(3-(1-((5-ethylthiazol-2-yl)amino)-1-oxopropan-2-yl)phenyl)pyridin-2-yl)-4-morpholinobut-2-enamide;
136) (S,E)-N-(3-cyano-5-(3-(1-((5-ethylthiazol-2-yl)amino)-1-oxopropan-2-yl)phenyl)pyridin-2-yl)-4-(4-methylpiperazin-1-yl)but-2-enamide;
137) (E)-N-(5-cyano-5'-(1-((5-ethylthiazol-2-yl)amino)-1-oxopropan-2-yl)-[3,3'-bipyridin]-6-yl)-4-(dimethylamino)but-2-enamide;
138) (E)-N-(3-cyano-5-(3-(1-((5-ethylthiazol-2-yl)amino)-1-oxobutan-2-yl)phenyl)pyridin-2-yl)-4-(dimethylamino)but-2-enamide;
139) (S)-2-(3-(5-cyano-6-(2-cyanoacetamido)pyridin-3-yl)phenyl)-N-(5-ethylthiazol-2-yl)propanamide;
140) N-(5-(6-(1-((5-methylthiazol-2-yl)amino)-1-oxopropan-2-yl)pyrazin-2-yl)pyridin-2-yl)acrylamide;
141) N-(2'-(1-((5-methylthiazol-2-yl)amino)-1-oxopropan-2-yl)-[3,4'-bipyridin]-6-yl)acrylamide;
142) N-(4-(3-(1-((5-methylthiazol-2-yl)amino)-1-oxopropan-2-yl)piperidin-1-yl)phenyl)acrylamide;
143) N-(5-(2-(1-((5-methylthiazol-2-yl)amino)-1-oxopropan-2-yl)thiazol-4-yl)pyridin-2-yl)acrylamide;
144) (S)-N-(3-cyclopropyl-5-(3-(1-((5-ethylthiazol-2-yl)amino)-1-oxopropan-2-yl)phenyl)pyridin-2-yl)acrylamide;
145) (S)-N-(3-((2-(dimethylamino)ethyl)(methyl)amino)-3'-(1-((5-ethylthiazol-2-yl)amino)-1-oxopropan-2-yl)-[1,1'-biphenyl]-4-yl)acrylamide;
146) (S)-N-(5-ethylthiazol-2-yl)-2-(3-(6-(vinylsulfonamido)pyridin-3-yl)phenyl)propanamide;
147) (S)-N-(3-(3-(1-((5-ethylthiazol-2-yl)amino)-1-oxopropan-2-yl)phenyl)-1-methyl-1H-pyrazol-5-yl)acrylamide;
148) (S)-N-(4-(3-(1-((5-ethylthiazol-2-yl)amino)-1-oxopropan-2-yl)phenyl)-1H-imidazol-2-yl)acrylamide; and
149) (S)-N-(4-(3-(1-((5-ethylthiazol-2-yl)amino)-1-oxopropan-2-yl)phenyl)thiazol-2-yl)acrylamide.

In a preferred embodiment, the present invention relates to a compound of Formula 2 below. 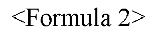 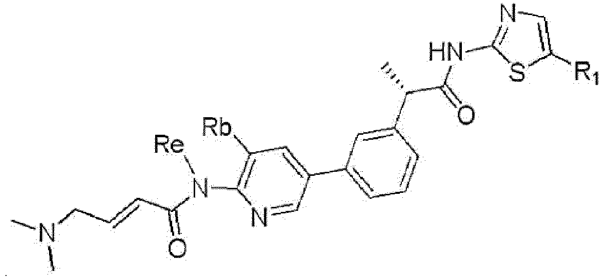 in the formula,
R1 is a linear C₁-C₃ alkyl unsubstituted or substituted with halogen; and
Rb is cyano or halo, and Re is H; or
Rb and Re are fused together with the pyridine linked to Rb and the nitrogen linked to Re to form 2,3-dihydro-1H-pyrrolo[2,3-b]pyridine.

In a preferred embodiment, the compound of Formula 2 is a compound selected from the group consisting of the following compounds.
(S,E)-4-(dimethylamino)-N-(3-fluoro-5-(3-(1-oxo-1-((5-(trifluoromethyl)thiazol-2-yl)amino)propan-2-yl)phenyl)pyridin-2-yl)but-2-enamide;
(S,E)-N-(3-cyano-5-(3-(1-oxo-1-((5-(trifluoromethyl)thiazol-2-yl)amino)propan-2-yl)phenyl)pyridin-2-yl)-4-(dimethylamino)but-2-enamide;
(S,E)-2-(3-(1-(4-(dimethylamino)but-2-enoyl)-2,3-dihydro-1H-pyrrolo[2,3-b]pyridin-5-yl)phenyl)-N-(5-(trifluoromethyl)thiazol-2-yl)propanamide;
(S,E)-N-(3-chloro-5-(3-(1-oxo-1-((5-(trifluoromethyl)thiazol-2-yl)amino)propan-2-yl)phenyl)pyridin-2-yl)-4-(dimethylamino)but-2-enamide;
(S,E)-N-(3-cyano-5-(3-(1-((5-ethylthiazol-2-yl)amino)-1-oxopropan-2-yl)phenyl)pyridin-2-yl)-4-(dimethylamino)but-2-enamide;
(S,E)-2-(3-(1-(4-(dimethylamino)but-2-enoyl)-2,3-dihydro-1H-pyrrolo[2,3-b]pyridin-5-yl)phenyl)-N-(5-ethylthiazol-2-yl)propanamide; and
(S,E)-4-(dimethylamino)-N-(5-(3-(1-((5-ethylthiazol-2-yl)amino)-1-oxopropan-2-yl)phenyl)-3-fluoropyridin-2-yl)but-2-enamide.

### Use in the prevention or treatment of cancer

The present invention relates to a pharmaceutical composition for preventing or treating cancer, wherein the pharmaceutical composition comprises a thiazole derivative or a pharmaceutically acceptable salt thereof.

In addition, the present invention relates to a method for treating cancer by administering a thiazole derivative or a pharmaceutically acceptable salt thereof to a subject to be treated.

The compound of the present invention or a pharmaceutically acceptable salt thereof inhibits the activity of CDK7, thereby exhibiting an effect of preventing or treating a proliferative disease, particularly cancer, associated therewith.

Specifically, the compound of the present invention or a pharmaceutically acceptable salt thereof phosphorylates the C-terminal domain (CTD) of RNA polymerase (RNAP) II to regulate the expression of oncogenes such as Myc, the expression of oncogenic transcription factors, and the expression of immune checkpoint proteins responsible for immune evasion, and inhibits the activity of CDK7, which regulates the cell cycle, thereby preventing the growth, survival, or proliferation of cancer cells, thereby exhibiting a preventive or therapeutic effect on cancer.

In addition, the compound of the present invention or a pharmaceutically acceptable salt thereof inhibits the activity of CDK7 to cause cytotoxicity through induction of apoptosis, thereby exhibiting a preventive or therapeutic effect on a proliferative disease, particularly cancer.

Therefore, the compound of the present invention or a pharmaceutically acceptable salt thereof can be usefully utilized as a preventive or therapeutic agent for cancer.

The "proliferative disease" may refer to cancer, benign neoplasm, angiogenesis, inflammatory disease, or auto-inflammatory disease.

The "cancer" refers to a malignant neoplasm, and exemplary cancers that can be treated with the compound of the present invention or a pharmaceutically acceptable salt thereof include: acoustic neuroma; adenocarcinoma; adrenal cancer; anal cancer; angiosarcoma (for example, lymphangiosarcoma, lymphangioendothelial sarcoma, hemangiosarcoma); appendix cancer; benign monoclonal gammopathy; biliary tract cancer (for example, cholangiocarcinoma); bladder cancer; breast cancer (for example, adenocarcinoma of the breast, papillary carcinoma of the breast, breast cancer (mammary cancer), medullary carcinoma of the breast); brain cancer (for example, meningioma, glioblastoma, glioma (for example, astrocytoma, oligodendroglioma), medulloblastoma); bronchial cancer; carcinoid tumor; cervical cancer (for example, cervical adenocarcinoma); choriocarcinoma; chordoma; craniopharyngioma; colorectal cancer (for example, colon cancer, rectal cancer, colorectal adenocarcinoma); connective tissue cancer; epithelial carcinoma; ependymoma; endothelial sarcoma (for example, Kaposi's sarcoma, multiple idiopathic hemorrhagic sarcoma); endometrial cancer (for example, uterine cancer, uterine sarcoma); esophageal cancer (for example, adenocarcinoma of the esophagus, Barrett's adenocarcinoma); Ewing's sarcoma; eye cancer (for example, intraocular melanoma, retinoblastoma); familial hypereosinophilia; gallbladder cancer; gastric cancer (for example, gastric adenocarcinoma); gastrointestinal stromal tumor (GIST); germ cell cancer; head and neck cancer (for example, squamous cell carcinoma of the head and neck); oral cancer (for example, oral squamous cell carcinoma); throat cancer (for example, laryngeal cancer, pharyngeal cancer, nasopharyngeal cancer, oropharyngeal cancer); hematopoietic cancer (for example, leukemia, such as acute lymphocytic leukemia (ALL) (for example, B cell ALL, T cell ALL), acute myeloid leukemia (AML) (for example, B cell AML, T cell AML), acute promyelocytic leukemia, chronic myeloid leukemia (CML) (for example, B cell CML, T cell CML), and chronic lymphocytic leukemia (CLL) (for example, B cell CLL, T cell CLL)); lymphoma, such as Hodgkin's lymphoma (HL) (for example, B cell HL, T cell HL) and non-Hodgkin's lymphoma (NHL) (for example, B cell NHL, such as diffuse large cell lymphoma (DLCL) (for example, diffuse large B cell lymphoma), follicular lymphoma, chronic lymphocytic leukemia/small lymphocytic lymphoma (CLL/SLL), mantle cell lymphoma (MCL), marginal zone B cell lymphoma (for example, mucosa-associated lymphoid tissue (MALT) lymphoma, nodular marginal zone B cell lymphoma, splenic marginal zone B cell lymphoma), primary mediastinal B cell lymphoma, Burkitt lymphoma, lymphoplasmacytic lymphoma (i.e., Waldenstrom macroglobulinemia), hairy cell leukemia (HCL), immunoblastic large cell lymphoma, precursor B-lymphoblastic lymphoma and primary central nervous system (CNS) lymphoma; and T cell NHL, such as precursor T-lymphoblastic lymphoma/leukemia, peripheral T cell lymphoma (PTCL) (for example, cutaneous T cell lymphoma (CTCL) (for example, mycosis fungoides, Sezary syndrome), angioimmunoblastic T cell lymphoma, extranodular natural killer T cell lymphoma, enteropathic T cell lymphoma, subcutaneous panniculitis-like T cell lymphoma, and anaplastic large cell lymphoma)); a mixture of one or more leukemias/lymphomas as described above; multiple myeloma (MM); heavy chain disease (for example, alpha chain disease, gamma chain disease, mu chain disease); hemangioblastoma; hypopharyngeal cancer; inflammatory myofibroblastic tumor; immune cell amyloidosis; kidney cancer (for example, Wilms' tumor also known as nephroblastoma, renal cell carcinoma); liver cancer (for example, hepatocellular cancer (HCC), malignant hepatocellular cancer); lung cancer (for example, bronchogenic carcinoma, small cell lung cancer (SCLC), non-small cell lung cancer (NSCLC), adenocarcinoma of the lung); leiomyosarcoma (LMS); mastocytosis (for example, systemic mastocytosis); muscle cancer; myelodysplastic syndrome (MDS); mesothelioma; myeloproliferative disorder (MPD) (for example, polycythemia vera (PV), essential thrombocythemia (ET), agnogenic myeloid metaplasia (AMM), also known as myelofibrosis (MF), chronic idiopathic myelofibrosis, chronic myeloid leukemia (CML), chronic neutrophilic leukemia (CNL), hypereosinophilic syndrome (HES)); neuroblastoma; neurofibroma (for example, neurofibromatosis type 1 or type 2 (NF), schwannomatosis); neuroendocrine cancer (for example, gastroenteropancreatic neuroendocrine tumor (GEP-NET), carcinoid tumor); osteosarcoma (for example, bone cancer); ovarian cancer (for example, cystadenocarcinoma, ovarian embryonic carcinoma, ovarian adenocarcinoma); papillary adenocarcinoma; pancreatic cancer (for example, pancreatic adenocarcinoma, intraductal papillary mucinous neoplasm (IPMN), islet cell tumor); penile cancer (for example, Paget's disease of the penis and scrotum); pineal tumor; primitive neuroectodermal tumor (PNT); plasma cell neoplasm; paraneoplastic syndrome; intraepithelial neoplasm; prostate cancer (for example, prostate adenocarcinoma); rectal cancer; rhabdomyosarcoma; salivary gland cancer; skin cancer (for example, squamous cell carcinoma (SCC), keratoacytoma (KA), melanoma, basal cell carcinoma (BCC)); small intestine cancer (for example, appendix cancer); soft tissue sarcoma (for example, malignant fibrous histiocytoma (MFH), liposarcoma, malignant peripheral nerve sheath tumor (MPNST), chondrosarcoma, fibrosarcoma, myxosarcoma); sebaceous gland carcinoma; large intestine cancer; sweat gland carcinoma; synovioma; testicular cancer (for example, seminoma, testicular embryonic carcinoma); thyroid cancer (for example, papillary carcinoma of the thyroid gland, papillary thyroid carcinoma (PTC), medullary thyroid cancer); urethral cancer; vaginal cancer; and vulvar cancer (for example, Paget's disease of the vulva), but are not limited thereto.

The terms "neoplasm" and "tumor" are used interchangeably herein and refer to an abnormal mass of tissue in which the growth of the mass exceeds that of normal tissue and is not coordinated with the growth of normal tissue. Neoplasms or tumors may be "benign" or "malignant" depending on the degree of cell differentiation (including morphology and functionality), growth rate, local invasion, and metastasis. "Benign neoplasms" are generally well differentiated, characterized by slower growth than malignant neoplasms, and remain localized at the site of origin. In addition, benign neoplasms do not have the ability to infiltrate, invade, or metastasize to distant sites. Exemplary benign neoplasms may include, but are not limited to, lipoma, chondroma, adenoma, soft fibroma, senile hemangioma, seborrheic keratosis, age spots, and sebaceous hyperplasia. In some cases, certain "benign" tumors may later lead to malignant neoplasms, which may result from additional genetic changes in a subpopulation of neoplastic cells of the tumor, and these tumors are "pre-malignant neoplasm." An exemplary pre-malignant neoplasm is a teratoma. In contrast, "malignant neoplasms" are generally poorly differentiated (anaplastic) and are characterized by rapid growth with progressive infiltration, invasion, and disruption of surrounding tissue. Moreover, malignant neoplasms generally have the ability to metastasize to distant sites.

The novel thiazole derivative according to the present invention not only have high selective inhibitory activity on the activity of CDK7 as compared to any compound known in the art, but also have reduced side effects and toxicity, which makes it possible to exert a remarkable preventive or therapeutic effect on proliferative diseases associated with CDK7, in particular cancer.

Hereinafter, the present invention will be described in more detail through the examples, but the following examples are for illustrative purposes only and are not intended to limit the scope of the present invention.

### [Example]

In order to confirm the efficacy of the compound of the present invention, the experiment was carried out as follows and the results were confirmed.

### [Example 1]

### CDK7 activity inhibitory effect

In order to confirm the CDK7 activity inhibition efficacy of the compound of the present invention, the level of CDK7 activity was measured when treated with the compound of the present invention using the ADP-Glo platform as follows.

Recombinant purified human CDK7 (ThermoFisher, PV3868) and ADP Glo Kinase Assay Kit (Promega, V9102) were used. CDK7 was diluted with IX kinase reaction buffer (40 mM Tris-Cl, pH 7.5, 20 mM MgCl₂, 0.1 mg/ml BSA and 50 µM DTT) and added to a 96 well plate (CDK7 final concentration per reaction: 50 ng). It was finally treated with the compound of the present invention to a 1% DMSO aqueous solution, and a substrate cocktail containing ATP (final concentration of 90 µM) and 0.2 µg/µl MBP (myelin basic protein) in a total of 25 µl reaction mass was added to a 96 well plate, thereby initiating the enzymatic reaction. After 2 hours of incubation (30 °C), an equal volume (25 µl per reaction) of ADP Glo was added and incubated at room temperature for 60 minutes (30 °C). Thereafter, kinase detection reagent (50 µl per reaction) was added and incubated at room temperature for 30 minutes (30 °C). The kinase activity was measured by the chemiluminescence method according to the ADP Glo Kinase Assay Kit instruction manual, and the CDK7 inhibitory activity of the compounds of the present invention was calculated. Analysis of the results of each compound was performed using Microsoft Excel, and IC₅₀ values were calculated by Prism software and are shown in Table 1 below.

**[Table 1]**

| Compound No. | CDK7 inhibition (IC₅₀ nM) | Compound No. | CDK7 inhibition (IC₅₀ nM) |
|---|---|---|---|
| 2 | 40.0 | 106 | 71.0 |
| 6 | 35.0 | 107 | 31.0 |
| 14 | 28.0 | 108 | 10.5 |
| 15 | 11.0 | 109 | 8.2 |
| 17 | 11.0 | 110 | 5.9 |
| 37 | 17.8 | 111 | 9.5 |
| 45 | 22.7 | 112 | 17.0 |
| 52 | 19.2 | 113 | 8.5 |
| 63 | 17.8 | 114 | 12.0 |
| 73 | 43.4 | 115 | 17.0 |
| 74 | 77.0 | 116 | 13.5 |
| 76 | 92.0 | 117 | 15.4 |
| 81 | 6.4 | 118 | 20.0 |
| 82 | 36.0 | 119 | 9.0 |
| 83 | 23.0 | 120 | 16.0 |
| 84 | 68.0 | 121 | 4.2 |
| 88 | 16.0 | 122 | 6.0 |
| 91 | 8.4 | 123 | 5.3 |
| 92 | 42.0 | 124 | 4.5 |
| 93 | 93.0 | 125 | 13.2 |
| 94 | 14.5 | 128 | 50.0 |
| 95 | 80.4 | 129 | 17.0 |
| 96 | 7.6 | 130 | 4.0 |
| 98 | 87.0 | 131 | 59.0 |
| 99 | 7.6 | 132 | 6.6 |
| 100 | 9.8 | 133 | 47.0 |
| 101 | 4.3 | 135 | 13.0 |
| 102 | 13.0 | 136 | 33.0 |
| 103 | 13.0 | 137 | 13.0 |
| 104 | 9.0 | 138 | 8.0 |

As shown in Table 1 above, it was confirmed that the compounds of the present invention exhibit an IC₅₀ of 100 nM or less, thereby having a high CDK7 inhibitory activity, and in particular, the compounds of Nos. 101, 121, 124, and 130 have an IC₅₀ value of 5.0 nM or less, thereby having a very high CDK7 inhibitory activity.

### [Example 2]

### CDK7 selective inhibitory effect

The level of CDK2, CDK5, and CDK7 inhibition was measured using a scanMAX kinase assay panel containing a set of 468 kinases including CDK2 and CDK5. The compounds of the present invention were screened at 1 µM, and the results are shown in Table 2 below.

**[Table 2]**

| Compound No. | Inhibition level (%) in 1 uM | | |
|---|---|---|---|
| | CDK2 | CDK5 | CDK7 |
| 101 | 26 | 22 | 99 |
| 121 | 20 | 30 | 99 |

As shown in Table 2 above, the compound of the present invention exhibited a very high inhibitory activity of 99% against CDK7 and low inhibitory activity against CDK2 and CDK5. From the above results, it can be seen that the compound of the present invention selectively inhibits CDK7.

### [Example 3]

### Gene expression inhibitory effect in cancer cells

### Example 3-1 : MDA-MB-468 cells

MDA-MB-468 cells are one of the triple-negative breast cancer (TNBC: negative for the expression of estrogen receptor, progesterone receptor and HER2) cells known as a malignant tumor that is difficult to diagnose at an early stage and has a high metastatic capacity, and these cells are used to test inhibition of proliferation and survival of breast cancer cells. In order to evaluate the efficacy of inhibiting the expression of genes related to cancer cell proliferation in MDA-MB-468 cells, the compounds of the present invention were tested at different concentrations (from 250 nM to 100 nM) as follows.

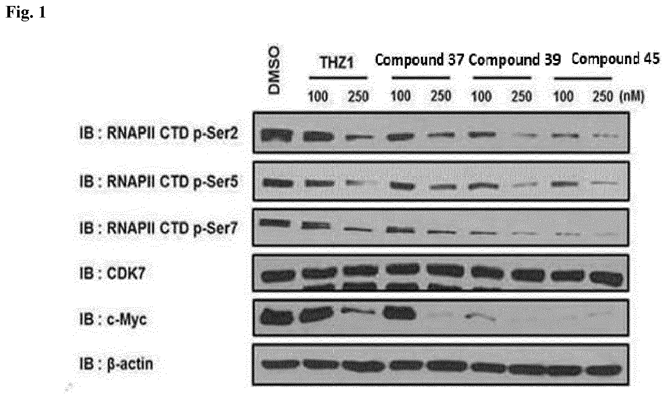

The cells were cultured in RPMI 1640 (Welgene) + 10% FBS (Gibco) + 1% penicillin/streptomycin (Gibco) and cultured at 37 °C in a humidified chamber in the presence of 5% CO₂. MDA-MB-468 cells cultured in a 6-well cell culture plate were treated with the compound of the present invention and incubated for 4 hours. Thereafter, proteins were extracted from the cells using RIPA (Radioimmunoprecipitation Assay) buffer containing protease and phosphatase inhibitors. The proteins were separated based on their molecular weight by SDS-PAGE and transferred to a nitrocellulose membrane. RNAPII CTD p-Ser2, RNAPII CTD p-Ser5, RNAPII CTD p-Ser7, CDK7, c-Myc and beta-actin antibodies were reacted with the membrane at 4 °C for 18 hours, and then the antibodies were reacted with ECL (high-sensitivity chemiluminescence). The light emission generated during the reaction was transmitted and developed on the X-ray film, and the results are shown in Fig. 1.

As a result, it was confirmed that the phosphorylation level of RNAPII CTD and the expression of c-Myc were greatly reduced by treatment with the compound according to the present invention. In addition, the compound of the present invention exhibited a higher effect as compared to THZ1, a CDK7 inhibitor used as a control group (Fig. 1).

### Example 3-2 : MDA-MB-231 cells

MDA-MB-231 cells are the triple-negative breast cancer (TNBC: negative for the expression of estrogen receptor, progesterone receptor and HER2) cells known as a malignant tumor that is difficult to diagnose at an early stage and has a high metastatic capacity. In order to evaluate the efficacy of inhibiting the expression of genes related to cancer cell proliferation in MDA-MB-231 cells, MDA-MB-231 cells were treated with the compounds of the present invention, which were tested as follows.

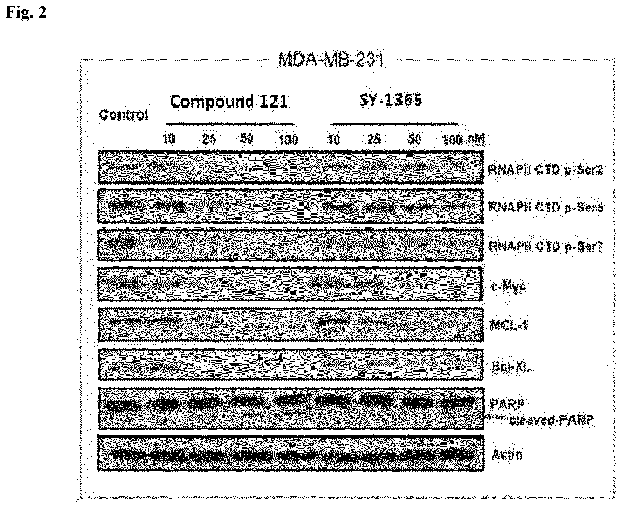

The cells were cultured in RPMI 1640 (Welgene) + 10% FBS (Gibco) + 1% penicillin/streptomycin (Gibco) and cultured at 37 °C in a humidified chamber in the presence of 5% CO₂. MDA-MB-231 cells cultured in a 6-well cell culture plate were treated with the compound of the present invention and incubated for 24 hours. Thereafter, proteins were extracted from the cells using RIPA (Radioimmunoprecipitation Assay) buffer containing protease and phosphatase inhibitors. The proteins were separated based on their molecular weight by SDS-PAGE and transferred to a nitrocellulose membrane. RNAPII CTD p-Ser2, RNAPII CTD p-Ser5, RNAPII CTD p-Ser7, c-Myc, MCL-1, Bcl-XL, PARP and beta-actin antibodies were reacted with the membrane at 4 °C for 18 hours, and then the antibodies were reacted with ECL (high-sensitivity chemiluminescence). The light emission generated during the reaction was transmitted and developed on the X-ray film, and the results are shown in Fig. 2.

As a result, it was confirmed that the phosphorylation level of RNAPII CTD and the expression of c-Myc, MCL-1 and Bcl-XL were greatly reduced by treatment with the compound according to the present invention. In addition, the compound of the present invention exhibited a higher effect as compared to SY-1365, a CDK7 inhibitor used as a control group (Fig. 2).

### Example 3-3 : HepG2 cells

HepG2 cells are human hepatocellular carcinoma cells. In order to evaluate the efficacy of inhibiting the expression of genes related to cancer cell proliferation in HepG2 cells, HepG2 cells were treated with the compounds of the present invention, which were tested as follows.

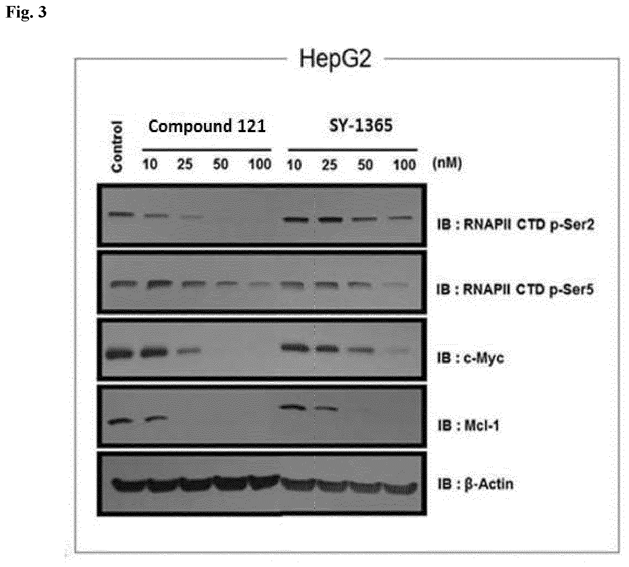

The cells were cultured in MEM (Welgene) + 10% FBS (Gibco) + 1% penicillin/streptomycin (Gibco) and cultured at 37 °C in a humidified chamber in the presence of 5% CO₂. HepG2 cells cultured in a 6-well cell culture plate were treated with the compound of the present invention and incubated for 4 hours. Thereafter, proteins were extracted from the cells using RIPA (Radioimmunoprecipitation Assay) buffer containing protease and phosphatase inhibitors. The proteins were separated based on their molecular weight by SDS-PAGE and transferred to a nitrocellulose membrane. RNAPII CTD p-Ser2, RNAPII CTD p-Ser5, c-Myc, MCL-1 and beta-actin antibodies were reacted with the membrane at 4 °C for 18 hours, and then the antibodies were reacted with ECL (high-sensitivity chemiluminescence). The light emission generated during the reaction was transmitted and developed on the X-ray film, and the results are shown in Fig. 3.

As a result, it was confirmed that the phosphorylation level of RNAPII CTD and the expression of c-Myc and MCL-1 were greatly reduced by treatment with the compound according to the present invention. In addition, the compound of the present invention exhibited a higher effect as compared to SY-1365, a CDK7 inhibitor used as a control group (Fig. 3).

### Example 3-4 : MV-4-11 cells

MV-4-11 cells are one of the acute myeloid leukemia cells with a Fms-like tyrosine kinase (FLT3) gene mutation. In order to evaluate the efficacy of inhibiting the expression of genes related to cancer cell proliferation in MV-4-11 cells, MV-4-11 cells were treated with the compounds of the present invention, which were tested as follows.

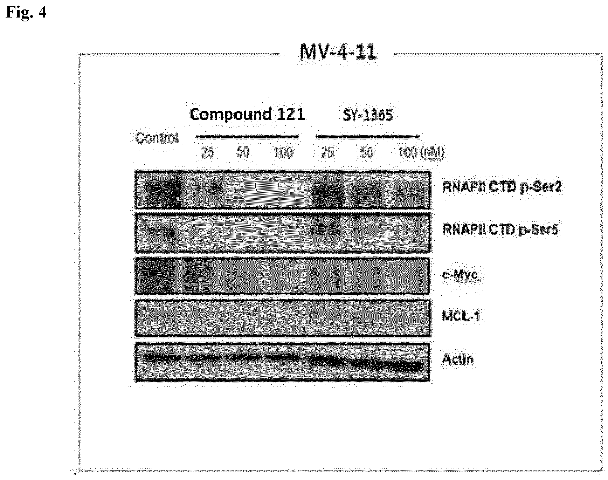

The cells were cultured in IMDM (Welgene) + 10% FBS (Gibco) + 1% penicillin/streptomycin (Gibco) and cultured at 37 °C in a humidified chamber in the presence of 5% CO₂. MV-4-11 cells cultured in a 6-well cell culture plate were treated with the compound of the present invention and incubated for 4 hours. Thereafter, proteins were extracted from the cells using RIPA (Radioimmunoprecipitation Assay) buffer containing protease and phosphatase inhibitors. The proteins were separated based on their molecular weight by SDS-PAGE and transferred to a nitrocellulose membrane. RNAPII CTD p-Ser2, RNAPII CTD p-Ser5, c-Myc, MCL-1 and beta-actin antibodies were reacted with the membrane at 4 °C for 18 hours, and then the antibodies were reacted with ECL (high-sensitivity chemiluminescence). The light emission generated during the reaction was transmitted and developed on the X-ray film, and the results are shown in Fig. 4.

As a result, it was confirmed that the phosphorylation level of RNAPII CTD and the expression of c-Myc and MCL-1 were greatly reduced by treatment with the compound according to the present invention. In addition, the compound of the present invention exhibited a higher effect as compared to SY-1365, a CDK7 inhibitor used as a control group (Fig. 4).

### Example 3-5 : MV-4-11 cells

MV-4-11 cells are one of the acute myeloid leukemia cells with a Fms-like tyrosine kinase (FLT3) gene mutation. In order to evaluate the efficacy of inhibiting the expression of genes related to cancer cell proliferation in MV-4-11 cells, MV-4-11 cells were treated with the compounds of the present invention, which were tested as follows.

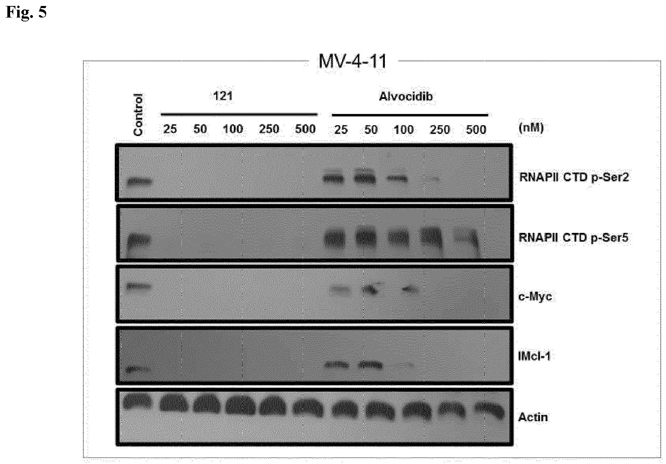

The cells were cultured in IMDM (Welgene) + 10% FBS (Gibco) + 1% penicillin/streptomycin (Gibco) and cultured at 37 °C in a humidified chamber in the presence of 5% CO₂. MV-4-11 cells cultured in a 6-well cell culture plate were treated with the compound of the present invention and cultured at 37 °C for 4 hours. Thereafter, proteins were extracted from the cells using RIPA (Radioimmunoprecipitation Assay) buffer containing protease and phosphatase inhibitors. The proteins were separated based on their molecular weight by SDS-PAGE and transferred to a nitrocellulose membrane. RNAPII CTD p-Ser2, RNAPII CTD p-Ser5, c-Myc, MCL-1 and beta-actin antibodies were reacted with the membrane at 4 °C for 18 hours, and then the antibodies were reacted with ECL (high-sensitivity chemiluminescence). The light emission generated during the reaction was transmitted and developed on the X-ray film, and the results are shown in Fig. 5.

As a result, it was confirmed that the phosphorylation level of RNAPII CTD and the expression of c-Myc were greatly reduced by treatment with the compound according to the present invention. In addition, the compound of the present invention exhibited a higher effect as compared to Alvocidib, a CDK9 inhibitor used as a control group (Fig. 5).

As confirmed above, it can be seen that the compound of the present invention inhibits the activity of CDK7 to reduce the phosphorylation of RNAPII CTD, which plays an important role in gene transcription process, and effectively inhibits Myc, MCL-1 and Bcl-XL associated with a proliferative disease, and thus, it has excellent activity for the treatment of a proliferative disease such as cancer.

### [Example 4]

### Cancer cell proliferation inhibitory effect

### Example 4-1 : MDA-MB-468 cells

MDA-MB-468 cells are one of the triple-negative breast cancer (TNBC: negative for the expression of estrogen receptor, progesterone receptor and HER2) cells known as a malignant tumor that is difficult to diagnose at an early stage and has a high metastatic capacity, and these cells are used to test inhibition of proliferation and survival of breast cancer cells. In order to evaluate the efficacy of inhibiting the proliferation of MDA-MB-468 cells, the compounds of the present invention were tested at different concentrations (from 1000 nM to 25 nM) as follows.

The cells were grown in RPMI 1640 (Welgene) + 10% FBS (Gibco) + 1% penicillin/streptomycin (Gibco) and cultured at 37 °C in a humidified chamber in the presence of 5% CO₂. MDA-MB-468 cells cultured in a 96-well cell culture plate were treated with the compound of the present invention and cultured for 72 hours. Thereafter, the antiproliferative effect of the compound was assayed by the MTT method using a tetrazolium salt.

Table 3 below shows the cell proliferation inhibition rate (%) at a compound concentration of 100 nM.

**[Table 3]**

| Compound No. | Inhibition rate (%) | Compound No. | Inhibition rate (%) |
|---|---|---|---|
| 1 | 54% | 33 | 59% |
| 2 | 50% | 36 | 64% |
| 4 | 56% | 37 | 60% |
| 6 | 66% | 38 | 64% |
| 11 | 53% | 39 | 67% |
| 14 | 74% | 40 | 64% |
| 15 | 73% | 45 | 59% |
| 16 | 53% | 47 | 72% |
| 17 | 70% | 49 | 67% |
| 22 | 72% | 50 | 66% |
| 25 | 71% | 52 | 72% |
| 27 | 74% | 53 | 51% |
| 28 | 74% | 61 | 73% |
| 29 | 75% | - | - |

Table 4 below shows the level to which the compounds of the present invention inhibit the proliferation of MDA-MB-468 cells as IC₅₀ (nM).

**[Table 4]**

| Compound No. | IC₅₀ (nM) | Compound No. | IC₅₀ (nM) |
|---|---|---|---|
| 2 | 104.0 | 104 | 44.0 |
| 6 | 86.0 | 108 | 31.0 |
| 14 | 63.0 | 109 | 31.0 |
| 15 | 46.0 | 110 | 74.0 |
| 17 | 26.0 | 111 | 32.0 |
| 37 | 40.0 | 112 | 59.0 |
| 45 | 9.5 | 113 | 35.0 |
| 63 | 20.0 | 114 | 56.0 |
| 73 | 35.0 | 115 | 75.0 |
| 74 | 65.0 | 116 | 65.0 |
| 81 | 39.0 | 117 | 56.0 |
| 82 | 32.0 | 118 | 47.0 |
| 83 | 21.0 | 119 | 44.0 |
| 88 | 54.0 | 120 | 51.0 |
| 91 | 38.0 | 121 | 14.0 |
| 92 | 52.0 | 122 | 34.0 |
| 93 | 38.0 | 123 | 24.0 |
| 94 | 59.0 | 124 | 40.0 |
| 95 | 57.0 | 125 | 33.0 |
| 96 | 68.0 | 130 | 7.0 |
| 99 | 23.0 | 132 | 32.0 |
| 100 | 56.0 | 135 | 48.0 |
| 101 | 30.0 | 137 | 47.0 |
| 102 | 57.0 | 138 | 100.0 |
| 103 | 68.0 | - | - |

As a result, experimental results showed that the compounds of the present invention inhibited the proliferation of cancer cells by at least 50% or more and inhibited the proliferation by 70% or more, confirming that the compounds of the present invention have the excellent cancer cell proliferation inhibitory effect. In addition, the compounds of the present invention exhibit an IC₅₀ value of 104.0 nM or less, and in particular, the compounds of Nos. 45, 121, and 130 exhibit an IC₅₀ value of 14.0 nM or less, confirming that they have a very excellent cancer cell proliferation inhibitory effect.

### Example 4-2 : MV-4-11 cells

MV-4-11 cells are one of the acute myeloid leukemia cells with a Fms-like tyrosine kinase (FLT3) gene mutation, and these cells are used to test inhibition of proliferation and survival of leukemia cells. In order to evaluate the ability of inhibiting the proliferation of MV-4-11 cells, the compounds of the present invention were tested at different concentrations (from 100 nM to 10 nM) as follows.

The cells were grown in IMDM (Welgene) + 10% FBS (Gibco) + 1% penicillin/streptomycin (Gibco) and cultured at 37 °C in a humidified chamber in the presence of 5% CO₂. MV-4-11 cells cultured in a 96-well cell culture plate were treated with the compound of the present invention and cultured for 72 hours. Thereafter, the antiproliferative effect of the compound was assayed by the CCK-8 method using a tetrazolium salt.

Table 5 below shows the cell proliferation inhibition rate (%) at a compound concentration of 100 nM.

**[Table 5]**

| Compound No. | Inhibition rate (%) | Compound No. | Inhibition rate (%) |
|---|---|---|---|
| 1 | 80% | 34 | 80% |
| 4 | 100% | 35 | 98% |
| 14 | 100% | 36 | 100% |
| 15 | 100% | 37 | 100% |
| 16 | 90% | 38 | 98% |
| 17 | 100% | 39 | 100% |
| 18 | 67% | 40 | 100% |
| 19 | 75% | 41 | 100% |
| 21 | 84% | 42 | 100% |
| 22 | 100% | 43 | 100% |
| 25 | 100% | 44 | 100% |
| 27 | 100% | 45 | 100% |
| 28 | 100% | 46 | 100% |
| 29 | 100% | 47 | 100% |
| 30 | 96% | 48 | 100% |
| 31 | 98% | 49 | 100% |
| 32 | 100% | 50 | 100% |
| 33 | 100% | - | - |

Table 6 below shows the level to which the compounds of the present invention inhibit the proliferation of MV-4-11 cells as IC₅₀ (nM).

**[Table 6]**

| Compound No. | IC₅₀ (nM) | Compound No. | IC₅₀ (nM) |
|---|---|---|---|
| 4 | 50.8 | 66 | 22.8 |
| 14 | 45.8 | 71 | 3.2 |
| 39 | 30.0 | 73 | 11.0 |
| 40 | 27.8 | 74 | 30.3 |
| 43 | 24.6 | 77 | 16.7 |
| 44 | 54.4 | 82 | 9.1 |
| 45 | 8.0 | 83 | 6.9 |
| 46 | 9.1 | 91 | 35.8 |
| 47 | 12.2 | 96 | 43.9 |
| 49 | 18.7 | 99 | 12.7 |
| 50 | 17.2 | 101 | 12.3 |
| 52 | 4.5 | 104 | 30.7 |
| 61 | 9.1 | 108 | 7.1 |
| 63 | 5.2 | 121 | 4.3 |

As a result, the compounds of the present invention exhibited an IC₅₀ value of 54.4 nM or less, and most of the compounds inhibited cell proliferation close to 100% and exhibited an IC₅₀ value of 20.0 nM or less, confirming that they have a very high leukemia cell proliferation inhibition efficacy.

### Example 4-3 : HepG2 cells

HepG2 cells are human hepatocellular carcinoma cells. In order to evaluate the efficacy of inhibiting the proliferation in HepG2 cells, HepG2 cells were treated with the compounds of the present invention, and the proliferation level was measured as follows.

The cells were cultured with a culture medium of MEM (Welgene) + 10% FBS (Gibco) + 1% penicillin/streptomycin (Gibco) at 37 °C in a humidified condition in the presence of 5% CO₂. HepG2 cells cultured in a 96-well cell culture plate were treated with the compound of the present invention and cultured for 72 hours. The antiproliferative effect of the compound was assayed by measuring cell viability by the CCK-8 method using a tetrazolium salt, and the IC₅₀ (nM) values are shown in Table 7 below.

**[Table 7]**

| Compound No. | IC₅₀ (nM) | Compound No. | IC₅₀ (nM) |
|---|---|---|---|
| 2 | 15.0 | 103 | 14.0 |
| 6 | 22.0 | 104 | 8.0 |
| 14 | 5.0 | 107 | 3.1 |
| 37 | 8.0 | 108 | 4.6 |
| 45 | 8.3 | 109 | 2.6 |
| 63 | 2.0 | 110 | 17.4 |
| 81 | 11.0 | 111 | 4.0 |
| 82 | 4.0 | 112 | 8.7 |
| 83 | 3.0 | 113 | 4.2 |
| 88 | 9.0 | 114 | 12.0 |
| 91 | 9.5 | 115 | 12.6 |
| 92 | 74.0 | 121 | 3.0 |
| 93 | 21.0 | 122 | 38.0 |
| 94 | 25.0 | 123 | 11.0 |
| 96 | 19.0 | 130 | 3.8 |
| 99 | 5.0 | 132 | 9.5 |
| 100 | 8.0 | 135 | 10.0 |
| 101 | 5.0 | 137 | 18.0 |
| 102 | 13.0 | 138 | 52.0 |

As a result, the compounds of the present invention exhibited an IC₅₀ value of 74.0 nM or less, and most of the compounds exhibited an IC₅₀ value of 15.0 nM or less, confirming that they have a very high liver cancer cell proliferation inhibition efficacy.

### [Example 5]

### Comparative experiment of inhibition of cancer cell proliferation

The proliferation effect of SY-1365, which is known to have a proliferation inhibitory effect on various carcinoma cells, and the compound of the present invention on various cancer cells was measured and compared as GI₅₀ (half maximal growth inhibitory concentration, the concentration at which cell proliferation is reduced by half).

Various carcinoma cells were cultured at 37 °C in a humidified condition in the presence of 5% CO₂. Each cell cultured in a 96-well cell culture plate was treated with the compound of the present invention and cultured for 72 hours. The antiproliferative effect of the compound was assayed by measuring cell viability by the CCK-8 method using a tetrazolium salt, and the GI₅₀ (nM) values are shown in Table 8 below.

**[Table 8]**

| | GI₅₀ (nM) | |
|---|---|---|
| | Compound 121 | SY-1365 |
| MDA-MB-231 | 6 | 70 |
| MDA-MB-468 | 4 | 18 |
| HepG2 | 14 | 19 |
| Hep3B | 9 | 36 |
| Huh-7 | 7 | 20 |

As a result, it was confirmed that the compound of the present invention had a more excellent antiproliferative effect on various carcinoma cells compared to SY-1365, a CDK7 inhibitor used as a control group.

### [Example 6]

### Proliferation inhibitory effect on various cancer cells

The compound of the present invention inhibits the gene transcription of cancer cells by inhibiting CDK7, which activates RNA polymerase (RNAP) II of cells, and thus has the effect of inhibiting the proliferation of various cancer diseases. In this example, in order to confirm the proliferation inhibitory effect on various cancer cells, Compound 121 was tested as follows.

The cells were cultured with a culture medium shown in Table 9 below at 37 °C in a humidified condition in the presence of 5% CO₂. Each cell cultured in a 96-well cell culture plate was treated with the compound of the present invention and cultured for 72 hours. The antiproliferative effect of the compound was assayed by measuring cell viability by the CCK-8 method using a tetrazolium salt, and the GI₅₀ (nM) values are shown in Table 9 below.

**[Table 9]**

| Cell line | Related disease | GI₅₀ (nM) | Culture medium |
|---|---|---|---|
| MDA-MB-468 | breast cancer | 4 | RPMI |
| MDA-MB-231 | breast cancer | 6 | RPMI |
| HCC70 | breast cancer | 11.2 | RPMI |
| MCF-7 | breast cancer | 15.1 | RPMI |
| HepG2 | liver cancer | 14 | MEM |
| Hep3B | liver cancer | 9 | MEM |
| Huh7 | liver cancer | 7 | MEM |
| OVCAR-3 | ovarian cancer | 4.8 | RPMI |
| SKOV-3 | ovarian cancer | 9.6 | McCoy's 5A |
| BxPC-3 | pancreatic cancer | 6.7 | RPMI |
| LNCaP | prostate cancer | 8 | RPMI |
| DU145 | prostate cancer | 11.5 | RPMI |
| A549 | lung cancer | 8.6 | RPMI |
| HCT116 | large intestine cancer | 4.0 | RPMI |
| HeLa | cervical cancer | 10.8 | MEM |
| HCC1937 | breast cancer | 14.6 | RPMI |
| T47D | breast cancer | 19.3 | RPMI |
| SK-BR-3 | breast cancer | 10.6 | RPMI |
| MOLM-13 | acute myeloid leukemia (AML) | 18.99 | RPMI |
| MOLM-14 | acute myeloid leukemia | 2.77 | RPMI |
| MV-4-11 | acute myeloid leukemia | 11.82 | IMDM |
| OCI-AML2 | acute myeloid leukemia | 7.74 | RPMI |
| THP-1 | acute myeloid leukemia | 1.95 | RPMI |
| U937 | acute myeloid leukemia | 2.43 | RPMI |
| KG-1 | acute myeloid leukemia | 29.04 | RPMI |
| SKM-1 | acute myeloid leukemia | 25.04 | RPMI |
| BDCM | acute myeloid leukemia | 2.68 | RPMI |
| NB4 | acute promyelocytic leukemia | 12.93 | RPMI |
| RCH-ACV | B cell acute lymphocytic leukemia | 4.1 | RPMI |
| TOM-1 | B cell acute lymphocytic leukemia | 4.36 | RPMI |
| NALM-20 | B cell acute lymphocytic leukemia | 18.56 | RPMI |
| KASUMI-2 | B cell acute lymphocytic leukemia | 6.26 | RPMI |
| CCRF-CEM | acute lymphocytic leukemia | 15.63 | RPMI |
| JURKAT | T cell acute lymphocytic leukemia | 17.47 | RPMI |
| MOLT4 | acute lymphocytic leukemia | 4.51 | RPMI |
| DAUDI | Burkitt lymphoma | 9.15 | RPMI |
| SR | immunoblastic large cell lymphoma | 5.83 | RPMI |
| K562 | chronic myeloid leukemia (CML) | 18.9 | RPMI |
| MEG-01 | chronic myeloid leukemia | 4 | RPMI |
| KU-812 | chronic myeloid leukemia | 3.64 | RPMI |
| RPMI-8226 | multiple myeloma | 16.43 | RPMI |
| MOLM/AZA-1 | acute myeloid leukemia | 13.19 | RPMI |
| MOLM/DEC-5 | acute myeloid leukemia | 14.91 | RPMI |
| THP/DEC-2 | acute myeloid leukemia | 3.6 | RPMI |
| MOLM/CYT | acute myeloid leukemia | 0.94 | RPMI |

As a result, the compound of the present invention exhibited a GI₅₀ value of less than 20.0 nM in most of cancer cells and a GI₅₀ value of less than 1.0 nM in cancer cells associated with acute myeloid leukemia.

Therefore, it can be seen that the compound of the present invention can selectively inhibit the activity of CDK7, which regulates the cell cycle and is responsible for the phosphorylation of RNAPII CTD, and inhibits the proliferation of various cancer cells.

### [Example 7]

### Cell cycle inhibitory effect on cancer cells

### Example 7-1 : MDA-MB-231 cells

MDA-MB-231 cells are the triple-negative breast cancer (TNBC: negative for the expression of estrogen receptor, progesterone receptor and HER2) cells known as a malignant tumor that is difficult to diagnose at an early stage and has a high metastatic capacity. In order to evaluate the efficacy of inhibiting the expression of cell cycle-related genes in MDA-MB-231 cells, MDA-MB-231 cells were treated with Compound 121 of the present invention, which was tested as follows.

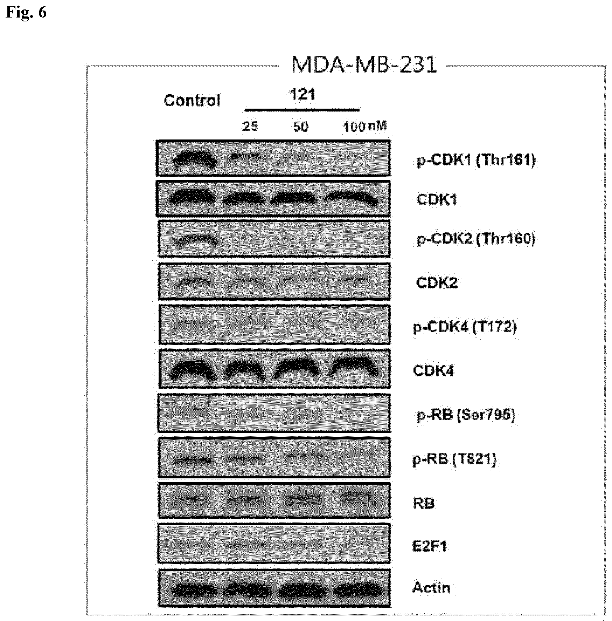

The cells were cultured in RPMI 1640 (Welgene) + 10% FBS (Gibco) + 1% penicillin/streptomycin (Gibco) and cultured at 37 °C in a humidified chamber in the presence of 5% CO₂. MDA-MB-231 cells cultured in a 6-well cell culture plate were treated with the compound of the present invention and cultured at 37 °C for 20 hours. Thereafter, proteins were extracted from the cells using RIPA (Radioimmunoprecipitation Assay) buffer containing protease and phosphatase inhibitors. The proteins were separated based on their molecular weight by SDS-PAGE and transferred to a nitrocellulose membrane. p-CDK1, CDK1, p-CDK2, CDK2, p-CDK4, CDK4, p-RB, RB, E2F1 and beta-actin antibodies were reacted with the membrane at 4 °C for 18 hours, and then the antibodies were reacted with ECL (high-sensitivity chemiluminescence). The light emission generated during the reaction was transmitted and developed on the X-ray film, and the results are shown in Fig. 6.

As a result, it was confirmed that the level of phosphorylation of CDK1, CDK2, and CDK4, and the level of phosphorylation of RB, which activate the cell cycle of cancer cells, were significantly reduced by treatment with the compound of the present invention (Fig. 6).

### Example 7-2 : MDA-MB-231 cells

MDA-MB-231 cells are the triple-negative breast cancer (TNBC: negative for the expression of estrogen receptor, progesterone receptor and HER2) cells known as a malignant tumor that is difficult to diagnose at an early stage and has a high metastatic capacity. In order to evaluate the efficacy of regulating the cell cycle in MDA-MB-231 cells, MDA-MB-231 cells were treated with Compound 121 of the present invention, which was tested as follows.

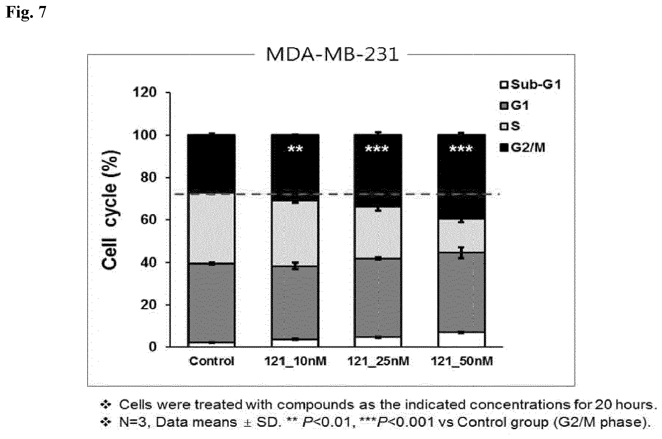

The cells were cultured in RPMI 1640 (Welgene) + 10% FBS (Gibco) + 1% penicillin/streptomycin (Gibco) and cultured at 37 °C in a humidified chamber in the presence of 5% CO₂. MDA-MB-231 cells cultured in a 6-well cell culture plate were treated with the compound of the present invention and cultured at 37 °C for 20 hours. Thereafter, the culture solution to which the compound was added was removed, and 70% ethanol was added to fix the cells at -20 °C for 18 hours. 70% ethanol was removed, and the cells were suspended with phosphate buffered saline, and then RNase (Sigma) and propidium isodide (Sigma) were added. The cell cycle was measured using a flow cytometer (ACEA bioscience) equipment, and the results are shown in Fig. 7.

As a result, it was confirmed that the cell cycle of cancer cells was significantly reduced due to the increase in the stagnation of the G2/M checkpoint, which is the stage of transition from the cell cycle of the cancer cell to mitosis by treatment with the compound of the present invention (Fig. 7).

### [Example 8]

### CDK7 occupancy rate and antitumor effect depending on exposure time in cancer cells

### Example 8-1 : MV4-11 cells

MV-4-11 cells are one of the acute myeloid leukemia cells with a Fms-like tyrosine kinase (FLT3) gene mutation. In order to evaluate the correlation between changes in CDK7 occupancy rate depending on exposure time and the cancer cell proliferation inhibitory effect in MV-4-11 cells, MV-4-11 cells were treated with Compound 121 of the present invention, which was tested as follows.

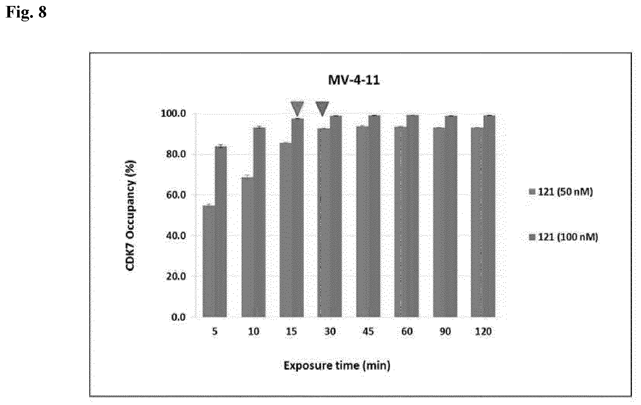

The cells were cultured in IMDM (Welgene) + 10% FBS (Gibco) + 1% penicillin/streptomycin (Gibco) and cultured at 37 °C in a humidified chamber in the presence of 5% CO₂. MV-4-11 cells cultured in a 6-well cell culture plate were treated with the compound of the present invention and cultured at 37 °C for 5 minutes to 120 minutes, respectively. Thereafter, proteins were extracted from the cells using a cell lysis solution (Cell Signaling). A biotin-labeled probe (ThermoFisher) was added to the extract and allowed to react at room temperature for 1 hour, and then transferred to a 96-well cell culture plate coated with streptavidin. A CDK7 antibody (Santa Cruz Biotechnology) was added and reacted at room temperature for 1 hour, and then a secondary antibody (Santa Cruz Biotechnology) was added and reacted at room temperature for 1 hour. After reacting with a fluorescent reagent (Cell Signaling), the absorbance was measured at a wavelength of 450 nm using a spectrometer (Urbans Bio), and the results are shown in Fig. 8.

As a result, it was confirmed that the CDK7 occupancy rate in cancer cells increased as the exposure time to the compound of the present invention increased, and it was confirmed that the exposure time-dependent CDK7 occupancy rate increased as the treatment concentration of the compound decreased. In addition, CDK7 occupancy rate of 80% or more was exhibited when the compound of the present invention was exposed for 15 minutes or more (Fig. 8).

### Example 8-2 : MV4-11 cells

MV-4-11 cells are one of the acute myeloid leukemia cells with a Fms-like tyrosine kinase (FLT3) gene mutation. In order to evaluate the efficacy of increasing the apoptosis activity of MV-4-11 cells depending on exposure time, MV-4-11 cells were treated with Compound 121 of the present invention, which was tested as follows.

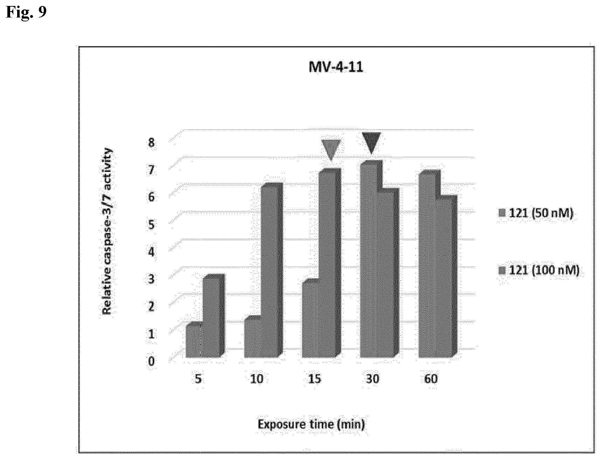

The cells were cultured in IMDM (Welgene) + 10% FBS (Gibco) + 1% penicillin/streptomycin (Gibco) and cultured at 37 °C in a humidified chamber in the presence of 5% CO₂. MV-4-11 cells cultured in a 6-well cell culture plate were treated with the compound of the present invention and cultured at 37 °C for 5 minutes to 60 minutes, respectively. Thereafter, the culture solution containing the compound was removed, replaced with a culture solution without the inhibitor, and cultured at 37 °C for 6 hours. Thereafter, Caspase-Glo^{®} 3/7 (Promega) analysis reagent was added and reacted at room temperature for 1 hour, and then the luminescence value was measured with a luminescence analyzer (Anthos Labtec), and the results are shown in Fig. 9.

As a result, it was confirmed that as the exposure time to the compound of the present invention increased, the activity of Caspase-3/7 inducing apoptosis of cancer cells was significantly increased. In addition, there was a clear correlation between the CDK7 occupancy rate, which was increased depending on the exposure time of the compound of the present invention, and the increased activity of Caspase-3/7 (Fig. 9).

### Example 8-3 : MV4-11 cells

MV-4-11 cells are one of the acute myeloid leukemia cells with a Fms-like tyrosine kinase (FLT3) gene mutation. In order to evaluate the efficacy of inhibiting the proliferation of MV-4-11 cells depending on exposure time, MV-4-11 cells were treated with Compound 121 of the present invention, which was tested as follows.

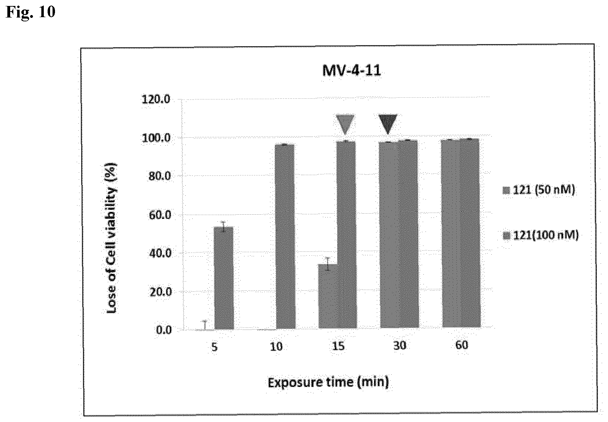

The cells were cultured in IMDM (Welgene) + 10% FBS (Gibco) + 1% penicillin/streptomycin (Gibco) and cultured at 37 °C in a humidified chamber in the presence of 5% CO₂. MV-4-11 cells cultured in a 96-well cell culture plate were treated with the compound of the present invention and cultured at 37 °C for 5 minutes to 60 minutes, respectively. Thereafter, the culture solution containing the compound was removed, replaced with a culture solution without the inhibitor, and cultured at 37 °C for 72 hours. Thereafter, CCK-8 (Biomax) analysis reagent using a tetrazolium salt was added, and then the absorbance was measured at a wavelength of 450 nm using a spectrometer (Urbans Bio), and the results are shown in Fig. 10.

As a result, it was confirmed that as the exposure time to the compound of the present invention increased, the growth inhibitory capacity of cancer cells significantly increased. In addition, it was confirmed that the compound of the present invention acts irreversibly, so that the cancer cell growth inhibition efficacy is maintained for a long time. Therefore, it was confirmed that the CDK7 occupancy rate showed a clear correlation with the increase in the activity of Caspase-3/7 and the growth inhibitory capacity, and it is related to the antitumor effect (Fig. 10).

### [Example 9]

### Cancer cell growth inhibitory effect in MOLM-13 orthotopic transplantation animal model

MOLM-13 cells showing cancer cell inhibitory effect were selected, and Compound 121 was tested as follows using an orthotopic transplantation model.

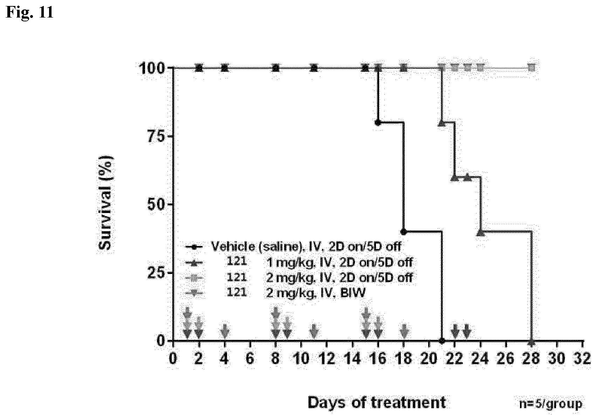

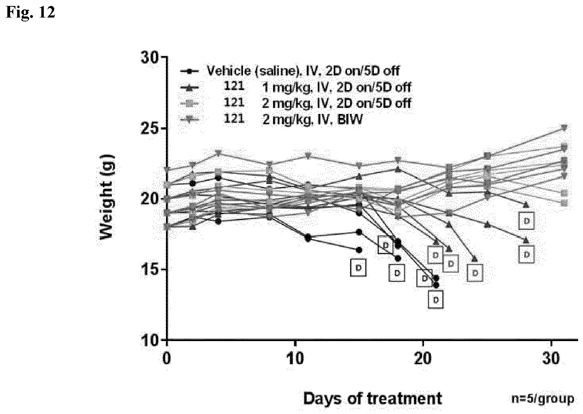

The animal model used for efficacy evaluation is an NSG (Jackson lab, #005557, USA) mouse lacking the IL-2 receptor gamma gene, in which the immune activity of T cells, B cells, and NK cells is suppressed, and the efficacy was evaluated in an orthotopic transplantation model of MOLM-13-GFP human cells. For the induction method, an orthotopic transplantation model was induced by injecting the cells in an amount of 5 × 10⁵ cells/200 ul/mouse into the tail vein route. Four test groups were G1 excipient control group vehicle (saline), G2 No. 121 1 mg/kg (2D on/ 5D off), G3 No. 121 2 mg/kg (2D on/ 5D off), and G4 No. 121 2 mg/kg (BIW), and 5 animals per group were set. From the day after cell injection, the tail vein administration of Compound 121 was started at each dose and regimen for 3 to 4 weeks. The survival rate and the change in body weight were evaluated until the end of the test, and the results are shown in Figs. 11 and 12.

As a result, an increase in the survival rate was confirmed in the group administered with Compound 121 of the present invention compared to the control group (Fig. 11). In addition, in the observation of the change in body weight, a constant amount of body weight gain was confirmed in the group administered with Compound 121 of the present invention compared to the control group (Fig. 12). Therefore, it can be seen that the compound of the present invention effectively inhibits tumor growth in an acute myeloid leukemia orthotopic transplantation model.

## Claims

1. A pharmaceutical composition for preventing or treating cancer, comprising a compound of Formula 1 below or a pharmaceutically acceptable salt thereof: 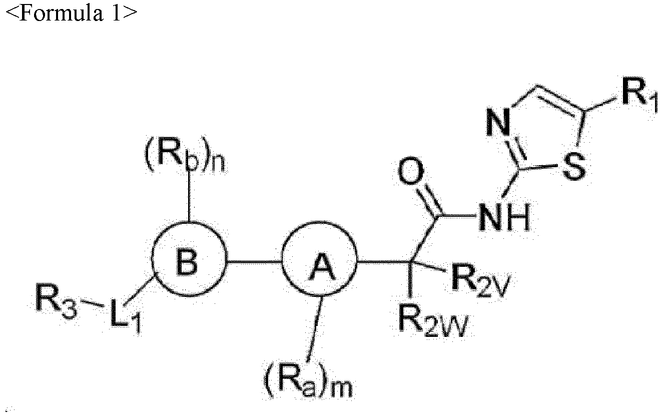 in the formula,
ring A is cycloalkyl, aryl, heteroaryl, or heterocycloalkyl;
ring B is cycloalkyl, aryl, heteroaryl, or heterocycloalkyl;
R₁ is H, halo, alkyl, cycloalkyl, heterocycloalkyl, aryl, heteroaryl, haloalkyl, cyano, - SR_{c}, -C(O)R_{c}, -C(O)OR_{c}, -C(O)N(R_{c})(R_{d}), -N(R_{c})(R_{d}), -(C(R_{g})₂)_{q}-N(R_{c})(R_{d}), -OR_{c}, or - (C(R_{g})₂)_{q}-OR_{c};
wherein R_{c} and R_{d} are each independently H, alkyl, cycloalkyl, aryl, heterocycloalkyl, or heteroaryl; or
R_{c} and R_{d} are taken together with the atoms to which they are attached to form an unsubstituted or substituted heterocycloalkyl;
q is an integer selected from 1 to 3; and
each R_{g} is independently H or alkyl;
R_{2V} and R_{2W} are each independently H, halo, hydroxy, alkyl, cycloalkyl, heterocycloalkyl, alkoxy, alkoxyalkyl, or amino; or
R_{2V} and R_{2W} are taken together with the atoms to which they are attached to form a cycloalkyl or heterocycloalkyl; and
L₁ is absent, -N(Rₑ)-, -^{∗}CH₂N(Rₑ)-, -^{∗}CH₂CH₂N(Rₑ)-, or alkylene, wherein ^{∗} is the position at which said L₁ is attached to ring B;
wherein Rₑ is H or alkyl; or Rₑ is bound to an atom of ring B to form a heteroaryl or heterocycloalkyl ring structure fused to ring B, which is unsubstituted or substituted;
R₃ is 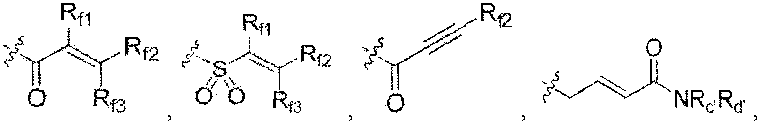 or 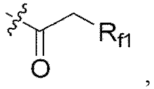
wherein R_{f1} is H, halo, alkyl, or cyano;
R_{f2} and R_{f3} are each independently H, halo, alkyl, cycloalkyl, heterocycloalkyl, heteroaryl, aryl, alkoxy, alkoxyalkyl, -N(R_{c'})(R_{d'}), or -(C(R_{g'})₂)_{q}-N(R_{c'})(R_{d'});
each R_{g'} is independently H or alkyl;
R_{c'} and R_{d'} are each independently H, alkyl, heterocycloalkyl, alkoxy, or alkoxyalkyl; or
R_{c'} and R_{d'} are taken together with the atoms to which they are attached to form an unsubstituted or substituted heterocycloalkyl,
each Rₐ is independently H, halo, hydroxyl, nitro, or cyano; or alkyl, alkoxy, alkoxyalkyl, amino, haloalkyl, aryl, heteroaryl, heterocycloalkyl, or cycloalkyl, which is unsubstituted or substituted; and
each R_{b} is independently H, halo, hydroxyl, nitro, or cyano; or alkyl, alkoxy, alkoxyalkyl, amino, haloalkyl, aryl, heteroaryl, heterocycloalkyl, cycloalkyl, or -NRₕRⱼ, which is unsubstituted or substituted;
wherein Rₕ and Rⱼ are each independently H, alkyl, -N(R_{h'})(R_{j'}), -(C(R_{g'})₂)_{q}-N(R_{h'})(R_{j'}), or heterocycloalkyl, and
R_{h'} and R_{j'} are each independently H or alkyl; or
R_{h'} and R_{j'} are taken together with the atoms to which they are attached to form an unsubstituted or substituted heterocycloalkyl; and
m and n are each independently an integer selected from 1 to 4.

2. The pharmaceutical composition for preventing or treating cancer according to claim 1, **characterized in that** ring A is 6- to 10-membered aryl; 5- or 6-membered heteroaryl containing 1 to 4 heteroatoms independently selected from the group consisting of N, O and S; or 5- or 6-membered heterocycloalkyl containing 1 to 4 heteroatoms independently selected from the group consisting of N, O and S.

3. The pharmaceutical composition for preventing or treating cancer according to claim 1, **characterized in that** ring A is phenyl, pyridinyl, pyrazinyl, pyrazolyl, thiophenyl, thiazolyl, or piperidinyl.

4. The pharmaceutical composition for preventing or treating cancer according to claim 1, **characterized in that** ring B is 6- to 10-membered aryl; 5- or 6-membered heteroaryl containing 1 to 4 heteroatoms independently selected from the group consisting of N, O and S; or 5- or 6-membered heterocycloalkyl containing 1 to 4 heteroatoms independently selected from the group consisting of N, O and S.

5. The pharmaceutical composition for preventing or treating cancer according to claim 1, **characterized in that** ring B is phenyl, pyridinyl, pyrimidinyl, pyrazinyl, pyridazinyl, pyrazolyl, imidazolyl, thiazolyl, tetrahydropyridinyl, piperidinyl, or piperazinyl.

6. The pharmaceutical composition for preventing or treating cancer according to claim 1, **characterized in that** R₁ is halo, C₁-C₆ alkyl, C₃-C₇ cycloalkyl, C₁-C₆ haloalkyl, cyano, - SR_{c}, -C(O)R_{c}, -C(O)OR_{c}, -N(R_{c})(R_{d}), -OR_{c}, or -C(R_{g})₂-OR_{c}; wherein R_{c} and R_{d} are each independently H or C₁-C₆ alkyl; and each R_{g} is independently H or C₁-C₆ alkyl.

7. The pharmaceutical composition for preventing or treating cancer according to claim 1, **characterized in that** R_{2V} and R_{2W} are each independently H, halo, or C₁-C₆ alkyl; or R_{2V} and R_{2W} are taken together with the atoms to which they are attached to form C₃-C₇ cycloalkyl.

8. The pharmaceutical composition for preventing or treating cancer according to claim 1, **characterized in that** L₁ is absent, -N(Rₑ)-, or -^{∗}CH₂N(Rₑ)-, wherein ^{∗} is the position at which said L₁ is attached to ring B;
wherein Rₑ is H or C₁-C₆ alkyl; or
Rₑ is bound to an atom of ring B to form, as a ring fused to ring B, 5- or 6-membered heteroaryl containing 1 to 4 heteroatoms independently selected from the group consisting of N, O and S; or 5- or 6-membered heterocycloalkyl containing 1 to 4 heteroatoms independently selected from the group consisting of N, O and S; wherein heteroaryl or heterocycloalkyl is unsubstituted or substituted with C₁-C₆ alkyl.

9. The pharmaceutical composition for preventing or treating cancer according to claim 1, **characterized in that** L₁ is absent, -N(Rₑ)-, or -^{∗}CH₂N(Rₑ)-, wherein ^{∗} is the position at which said L₁ is attached to ring B;
wherein Rₑ is H or C₁-C₆ alkyl; or
Rₑ is bound to an atom of ring B to form, as a ring fused to ring B, pyrrolidine or pyrrole, wherein pyrrolidine or pyrrole is unsubstituted or substituted with C₁-C₆ alkyl.

10. The pharmaceutical composition for preventing or treating cancer according to claim 1, **characterized in that**
R₃ is 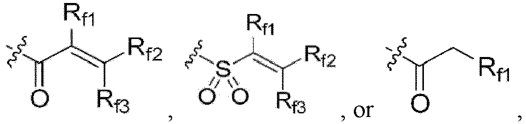 wherein R_{f1} is H, C₁-C₆ alkyl, or cyano; and
R_{f2} and R_{f3} are each independently H, C₁-C₆ alkyl, C₃-C₇ cycloalkyl, -N(R_{c'})(R_{d'}), or - (CH₂)N(R_{c'})(R_{d'});
wherein R_{c'} and R_{d'} are each independently H or C₁-C₆ alkyl; or
R_{c'} and R_{d'} are taken together with the atoms to which they are attached to form 4- to 7-membered heterocycloalkyl containing 1 to 3 heteroatoms independently selected from the group consisting of N, O and S, wherein heterocycloalkyl is unsubstituted or substituted with halo, hydroxy, C₁-C₆ alkyl, or C₁-C₆ alkoxy-C₁-C₆ alkyl.

11. The pharmaceutical composition for preventing or treating cancer according to claim 1, **characterized in that**
R₃ is 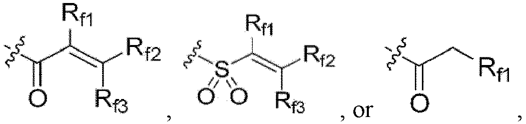 wherein R_{f1} is H, C₁-C₆ alkyl, or cyano; and
R_{f2} and R_{f3} are each independently H, C₁-C₆ alkyl, C₃-C₇ cycloalkyl, -N(CH₃)₂, or - (CH₂)N(R_{c}')(R_{d}');
wherein R_{c'} and R_{d'} are each independently C₁-C₆ alkyl; or
R_{c'} and R_{d'} are taken together with the atoms to which they are attached to form azetidinyl; piperidinyl; morpholinyl; piperazinyl unsubstituted or substituted with C₁-C₆ alkyl; 2,5-diazabicyclo[2.2.1]heptanyl unsubstituted or substituted with C₁-C₆ alkyl; 2-oxa-5-azabicyclo[2.2.1]heptanyl; or pyrrolidinyl unsubstituted or substituted with halo, hydroxy, or C₁-C₆ alkoxy-C₁-C₆ alkyl.

12. The pharmaceutical composition for preventing or treating cancer according to claim 1, **characterized in that** each Rₐ is independently H, halo, or C₁-C₆ alkyl; and
each R_{b} is independently H, halo, C₁-C₆ alkyl, C₃-C₇ cycloalkyl, C₁-C₆ alkoxy, cyano, C₁-C₆ haloalkyl, -NRₕRⱼ, or 5- to 6-membered heterocycloalkyl containing 1 to 4 heteroatoms independently selected from the group consisting of N, O and S, wherein heterocycloalkyl is unsubstituted or substituted with C₁-C₆ alkyl,
wherein Rₕ and Rⱼ are each independently C₁-C₆ alkyl or -CH₂CH₂N(R_{h'})(R_{j'}); and
R_{h'} and R_{j'} are each independently H or C₁-C₆ alkyl.

13. The pharmaceutical composition for preventing or treating cancer according to claim 1, **characterized in that** each Rₐ is independently H, halo, or C₁-C₆ alkyl; and
each R_{b} is independently H, halo, C₁-C₆ alkyl, C₃-C₇ cycloalkyl, C₁-C₆ alkoxy, cyano, C₁-C₆ haloalkyl, -N(CH₃)-CH₂CH₂-N(CH₃)₂, or piperazinyl unsubstituted or substituted with C₁-C₆ alkyl.

14. The pharmaceutical composition for preventing or treating cancer according to claim 1, **characterized in that** m and n are each 1.

15. The pharmaceutical composition for preventing or treating cancer according to claim 1, **characterized in that** the compound of Formula 1 is selected from the group consisting of the following compounds:
1) N-(5-(3-(1-((5-methylthiazol-2-yl)amino)-1-oxopropan-2-yl)phenyl)pyridin-2-yl)acrylamide;
2) N-(5-(3-(1-((5-cyclopropylthiazol-2-yl)amino)-1-oxopropan-2-yl)phenyl)pyridin-2-yl)acrylamide;
3) (R)-N-(5-(3-(1-((5-cyclopropylthiazol-2-yl)amino)-1-oxopropan-2-yl)phenyl)pyridin-2-yl)acrylamide;
4) N-(5-(3-(1-((5-ethylthiazol-2-yl)amino)-1-oxopropan-2-yl)phenyl)pyridin-2-yl)acrylamide;
5) (E)-4-(dimethylamino)-N-(5-(3-(1-((5-methylthiazol-2-yl)amino)-1-oxopropan-2-yl)phenyl)pyridin-2-yl)but-2-enamide;
6) (E)-N-(5-(3-(1-((5-cyclopropylthiazol-2-yl)amino)-1-oxopropan-2-yl)phenyl)pyridin-2-yl)-4-(dimethylamino)but-2-enamide;
7) (E)-4-(dimethylamino)-N-(5-(3-(1-((5-methylthiazol-2-yl)amino-1-oxopropan-2-yl)phenyl)pyridin-2-yl)but-2-enamide hydrochloride;
8) N-(3-fluoro-3'-(1-((5-methylthiazol-2-yl)amino-1-oxopropan-2-yl)-[1,1'-biphenyl]-4-yl)acrylamide;
9) 2-(3-(1-acryloylindolin-5-yl)phenyl)-N-(5-methylthiazol-2-yl)propanamide;
10) (E)-N-(5-(3-(1,1-difluoro-2-((5-methylthiazol-2-yl)amino)-2-oxoethyl)phenyl)pyridin-2-yl)-4-(dimethylamino)but-2-enamide;
11) N-(6-(3-(1-((5-cyanothiazol-2-yl)amino)-1-oxopropan-2-yl)-4-fluorophenyl)pyridazin-3 -yl)acrylamide;
12) 2-(3-(6-acrylamidopyndazin-3-yl)phenyl)-N-(5-cyanothiazol-2-yl)-3-methylbutanamide;
13) N-(6-(5-(1-((5-cyanothiazol-2-yl)amino)-1-oxopropan-2-yl)-2-fluorophenyl)pyridazin-3 -yl)acrylamide;
14) (S)-N-(5-(3-(1-(5-cyclopropylthiazol-2-yl)amino)-1-oxopropan-2-yl)phenyl)pyridin-2-yl)acrylamide;
15) (S,E)-N-(5-(3-(1-(5-cyclopropylthiazol-2-yl)amino)-1-oxopropan-2-yl)phenyl)pyridin-2-yl)-4-(dimethylamino)but-2-enamide;
16) (S)-N-(6-(3-(1-(5-cyclopropylthiazol-2-yl)amino)-1-oxopropan-2-yl)phenyl)pyrazin-2-yl)acrylamide;
17) (S,E)-4-(dimethylamino)-N-(5-(3-(1-((5-ethylthiazol-2-yl)amino)-1-oxopropan-2-yl)phenyl)pyridin-2-yl)but-2-enamide;
18) (S)-N-(5-(3-(1-((5-cyanothiazol-2-yl)amino)-1-oxopropan-2-yl)phenyl)pyridin-2-yl)acrylamide;
19) (S)-2-(3-(6-acrylamidopyridin-3-yl)phenyl)-N-(5-ethylthiazol-2-yl)butanamide;
20) (S)-2-(4'-acrylamido-3'-fluoro-[1,1'-biphenyl]-3-yl)-N-(5-ethylthiazol-2-yl)butanamide;
21) (S,E)-N-(5-(3-(1-((5-cyanothiazol-2-yl)amino)-1-oxopropan-2-yl)phenyl)pyridin-2-yl)-4-(dimethylamino)but-2-enamide;
22) (S)-N-(5-(3-(1-(5-cyclopropylthiazol-2-yl)amino)-1-oxopropan-2-yl)phenyl)pyrazin-2-yl)acrylamide;
23) (S)-N-(3'-(1-((5-cyclopropylthiazol-2-yl)amino)-1-oxopropan-2-yl)-[1,1'-biphenyl]-4-yl)acrylamide;
24) (S)-N-(3'-(1-((5-cyclopropylthiazol-2-yl)amino)-1-oxopropan-2-yl)-3-fluoro-[1,1'-biphenyl]-4-yl)acrylamide;
25) (S)-N-(5-(3-(1-((5-cyanothiazol-2-yl)amino)-1-oxopropan-2-yl)phenyl)pyrazin-2-yl)acrylamide;
26) (S)-N-(6-(3-(1-((5-cyanothiazol-2-yl)amino)-1-oxopropan-2-yl)phenyl)pyrazin-2-yl)acrylamide;
27) (S)-N-(5-(3-(1-(5-cyclopropylthiazol-2-yl)amino)-1-oxopropan-2-yl)phenyl)pyrimidin-2-yl)acrylamide;
28) (S)-N-(6-(3-(1-(5-cyclopropylthiazol-2-yl)amino)-1-oxopropan-2-yl)phenyl)pyridazin-3-yl)acrylamide;
29) (S)-N-(5-(3-(1-(5-cyclopropylthiazol-2-yl)amino)-1-oxopropan-2-yl)phenyl)-3-fluoropyridin-2-yl)acrylamide;
30) (S)-N-(3-cyano-3'-(1-((5-ethylthiazol-2-yl)amino)-1-oxopropan-2-yl)-[1,1'-biphenyl]-4-yl)acrylamide;
31) (S)-N-(5-(3-(1-((5-ethylthiazol-2-yl)amino)-1-oxopropan-2-yl)phenyl)-6-fluoropyridin-2-yl)acrylamide;
32) (S)-N-(6-(3-(1-((5-cyanothiazol-2-yl)amino)-1-oxopropan-2-yl)phenyl)pyridazin-3-yl)acrylamide;
33) (S)-N-(5-(3-(1-((5-cyanothiazol-2-yl)amino)-l-oxopropan-2-yl)phenyl)-3-fluoropyridin-2-yl)acrylamide;
34) (S)-N-(5-(3-(1-((5-cyanothiazol-2-yl)amino)-1-oxopropan-2-yl)phenyl)-3-methylpyrazin-2-yl)acrylamide;
35) (S)-N-(5-(3-(1-((5-isopropylthiazol-2-yl)amino)-1-oxopropan-2-yl)phenyl)pyridin-2-yl)acrylamide;
36) (S)-N-(5-(3-(1-((5-isopropylthiazol-2-yl)amino)-1-oxopropan-2-yl)phenyl)pyrazin-2-yl)acrylamide;
37) (S)-N-(5-(3-(1-oxo-1-((5-(trifluoromethyl)thiazol-2-yl)amino)propan-2-yl)phenyl)pyrazin-2-yl)acrylamide;
38) (S)-N-(5-(3-(1-oxo-1-((5-(trifluoromethyl)thiazol-2-yl)amino)propan-2-yl)phenyl)pyridin-2-yl)acrylamide;
39) (S)-N-(6-(3-(1-oxo-1-((5-(trifluoromethyl)thiazol-2-yl)amino)propan-2-yl)phenyl)pyridazin-3-yl)acrylamide;
40) (S)-N-(6-(3-(1-((5-isopropylthiazol-2-yl)amino)-1-oxopropan-2-yl)phenyl)pyridazin-3-yl)acrylamide;
41) N-(5'-(1-((5-cyanothiazol-2-yl)amino)-1-oxopropan-2-yl)-[3,3'-bipyridin]-6-yl)acrylamide;
42) N-(4-(1-((5-cyanothiazol-2-yl)amino)-1-oxopropan-2-yl)-[2,3'-bipyridin]-6'-yl)acrylamide;
43) N-(5-(5-(1-((5-cyclopropylthiazol-2-yl)amino)-1-oxopropan-2-yl)pyridin-3-yl)pyrazin-2-yl)acrylamide;
44) N-(5-(5-(1-((5-cyanothiazol-2-yl)amino)-1-oxopropan-2-yl)pyridin-3-yl)pyrazin-2-yl)acrylamide;
45) N-(5-(5-(1-oxo-1-((5-(trifluoromethyl)thiazol-2-yl)amino)propan-2-yl)pyridin-3-yl)pyrazin-2-yl)acrylamide;
46) N-(5-(5-(1-((5-ethylthiazol-2-yl)amino)-1-oxopropan-2-yl)pyridin-3-yl)pyrazin-2-yl)acrylamide;
47) N-(5-(5-(1-((5-isopropylthiazol-2-yl)amino)-1-oxopropan-2-yl)pyridin-3-yl)pyrazin-2-yl)acrylamide;
48) N-(5-(5-(1-((5-acetylthiazol-2-yl)amino)-1-oxopropan-2-yl)pyridin-3-yl)pyrazin-2-yl)acrylamide;
49) N-(6-(5-(1-oxo-1-((5-(trifluoromethyl)thiazol-2-yl)amino)propan-2-yl)pyridin-3-yl)pyridazin-3-yl)acrylamide;
50) N-(4-(1-((5-cyclopropylthiazol-2-yl)amino)-1-oxopropan-2-yl)-[2,3'-bipyridin]-6'-yl)acrylamide;
51) (R)-N-(5-(5-(1-oxo-1-((5-(trifluoromethyl)thiazol-2-yl)amino)propan-2-yl)pyridin-3-yl)pyrazin-2-yl)acrylamide;
52) (S)-N-(5-(5-(1-oxo-1-((5-(trifluoromethyl)thiazol-2-yl)amino)propan-2-yl)pyridin-3-yl)pyrazin-2-yl)acrylamide;
53) N-(5-(5-(1-oxo-1-((5-(trifluoromethyl)thiazol-2-yl)amino)propan-2-yl)thiophen-3-yl)pyridin-2-yl)acrylamide;
54) N-(5-(3-(1-((5-cyclopropylthiazol-2-yl)amino)-1-oxopropan-2-yl)-5-fluorophenyl)pyridin-2-yl)acrylamide;
55) N-(5-(5-(2-methyl-1-oxo-1-((5-(trifluoromethyl)thiazol-2-yl)amino)propan-2-yl)pyridin-3-yl)pyrazin-2-yl)acrylamide;
56) (S)-2-(3-(5-(2-cyanoacetamido)pyrazin-2-yl)phenyl)-N-(5-(trifluoromethyl)thiazol-2-yl)propanamide;
57) N-(5-(5-(1-oxo-1-((5-(trifluoromethyl)thiazol-2-yl)amino)propan-2-yl)thiophen-3-yl)pyrazin-2-yl)acrylamide;
58) (S)-2-(3-(5-(2-cyanoacetamido)pyrazin-2-yl)phenyl)-N-(5-cyanothiazol-2-yl)propanamide;
59) N-(5-(3-methyl-1-(1-oxo-1-((5-(tnfluoromethyl)thiazol-2-yl)amino)propan-2-yl)-1H-pyrazol-4-yl)pyridin-2-yl)acrylamide;
60) 2-(1'-acryloyl-1',2',3',6'-tetrahydro-[3,4'-bipyridin]-5-yl)-N-(5-cyanothiazol-2-yl)propanamide;
61) N-(5-(5-(1-((5-chlorothiazol-2-yl)amino)-1-oxopropan-2-yl)pyridin-3-yl)pyrazin-2-yl)acrylamide;
62) (S)-N-(6-(3-(1-((5-cyclopropylthiazol-2-yl)amino)-1-oxopropan-2-yl)phenyl)pyridin-3-yl)acrylamide;
63) (E)-4-(dimethylamino)-N-(5-(5-(1-oxo-1-((5-(trifluoromethyl)thiazol-2-yl)amino)propan-2-yl)pyridin-3-yl)pyrazin-2-yl)but-2-enamide;
64) 2-(5-(5-(2-cyanoacetamido)pyrazin-2-yl)pyridin-3-yl)-N-(5-(trifluoromethyl)thiazol-2-yl)propanamide;
65) (E)-2-cyano-3-cyclopropyl-N-(5-(5-(1-oxo-1-((5-(trifluoromethyl)thiazol-2-yl)amino)propan-2-yl)pyridin-3-yl)pyrazin-2-yl)acrylamide;
66) N-(5-(5-(1-((5-methoxythiazol-2-yl)amino)-1-oxopropan-2-yl)pyridin-3-yl)pyrazin-2-yl)acrylamide;
67) N-(5-(5-(1-((5-fluorothiazol-2-yl)amino)-1-oxopropan-2-yl)pyridin-3-yl)pyrazin-2-yl)acrylamide;
68) N-(2-(4-methylpiperazin-1-yl)-4-(5-(1-oxo-1-((5-(trifluoromethyl)thiazol-2-yl)amino)propan-2-yl)pyridin-3-yl)phenyl)acrylamide;
69) (E)-2-cyano-3-(dimethylamino)-N-(5-(5-(1-oxo-1-((5-(trifluoromethyl)thiazol-2-yl)amino)propan-2-yl)pyridin-3-yl)pyrazin-2-yl)acrylamide;
70) N-(1-(5-(1-oxo-1-((5-(trifluoromethyl)thiazol-2-yl)amino)propan-2-yl)pyridin-3-yl)piperidin-4-yl)acrylamide;
71) N-(5-(5-(1-((5-(methylthio)thiazol-2-yl)amino)-1-oxopropan-2-yl)pyridin-3-yl)pyrazin-2-yl)acrylamide;
72) ethyl 2-(2-(5-(5-acrylamidopyrazin-2-yl)pyridin-3-yl)propanamido)thiazole-5-carboxylate;
73) (E)-N-(5-(5-(1-((5-chlorothiazol-2-yl)amino)-1-oxopropan-2-yl)pyridin-3-yl)pyrazin-2-yl)-4-(dimethylamino)but-2-enamide;
74) (E)-4-morpholino-N-(5-(5-(1-oxo-1-((5-(trifluoromethyl)thiazol-2-yl)amino)propan-2-yl)pyridin-3-yl)pyrazin-2-yl)but-2-enamide;
75) (E)-N-(5-(5-(1-oxo-1-((5-(trifluoromethyl)thiazol-2-yl)amino)propan-2-yl)pyridin-3-yl)pyrazin-2-yl)but-2-enamide;
76) N-(5-(5-(1-((5-chlorothiazol-2-yl)amino)-1-oxopropan-2-yl)thiophen-3-yl)pyridin-2-yl)acrylamide;
77) N-(5-(5-(1-((5-ethylthiazol-2-yl)amino)-1-oxopropan-2-yl)thiophen-3-yl)pyridin-2-yl)acrylamide;
78) N-(5-(5-(1-((5-acetylthiazol-2-yl)amino)-l-oxopropan-2-yl)thiophen-3-yl)pyridin-2-yl)acrylamide;
79) 2-(5-(5-propionamidopyrazin-2-yl)pyridin-3-yl)-N-(5-(trifluoromethyl)thiazol-2-yl)propanamide;
80) (E)-4-(5-methyl-2,5-diazabicyclo[2.2.1]heptan-2-yl)-N-(5-(5-(1-oxo-1-((5-(trifluoromethyl)thiazol-2-yl)amino)propan-2-yl)pyridin-3-yl)pyrazin-2-yl)but-2-enamide;
81) (S,E)-4-(dimethylamino)-N-(5-(3-(1-oxo-1-((5-(trifluoromethyl)thiazol-2-yl)amino)propan-2-yl)phenyl)pyrazin-2-yl)but-2-enamide;
82) N-(5'-(1-oxo-1-((5-(trifluoromethyl)thiazol-2-yl)amino)propan-2-yl)-[3,3'-bipyridin]-6-yl)acrylamide;
83) (E)-4-(2-oxa-5-azabicyclo[2.2.1]heptan-5-yl)-N-(5-(5-(1-oxo-1-((5-(trifluoromethyl)thiazol-2-yl)amino)propan-2-yl)pyridin-3-yl)pyrazin-2-yl)but-2-enamide;
84) N-(4-fluoro-5'-(1-oxo-1-((5-(trifluoromethyl)thiazol-2-yl)amino)propan-2-yl)-[3,3'-bipyridin]-6-yl)acrylamide;
85) 2-(5-(4-acryloylpiperazin-1-yl)pyridin-3-yl)-N-(5-(trifluoromethyl)thiazol-2-yl)propanamide;
86) 2-(5-(4-acryloylpiperazin-1-yl)pyridin-3-yl)-N-(5-ethylthiazol-2-yl)propanamide;
87) N-(6-(5-(1-((5-ethylthiazol-2-yl)amino)-1-oxopropan-2-yl)thiophen-3-yl)pyridin-3-yl)acrylamide;
88) N-(5-cyano-5'-(1-oxo-1-((5-(trifluoromethyl)thiazol-2-yl)amino)propan-2-yl)-[3,3'-bipyridin]-6-yl)acrylamide;
89) N-((5'-(1-oxo-1-((5-(trifluoromethyl)thiazol-2-yl)amino)propan-2-yl)-[3,3'-bipyridin]-6-yl)methyl)acrylamide;
90) N-(5'-(1-oxo-1-((5-(trifluoromethyl)thiazol-2-yl)amino)propan-2-yl)-[2,3'-bipyridin]-5-yl)acrylamide;
91) (S,E)-4-(dimethylamino)-N-(5-(3-(1-oxo-1-((5-(trifluoromethyl)thiazol-2-yl)amino)propan-2-yl)phenyl)pyrazin-2-yl)but-2-enamide;
92) (S,E)-4-morpholino- N-(5-(3-(1-oxo-1-((5-(trifluoromethyl)thiazol-2-yl)amino)propan-2-yl)phenyl)pyrazin-2-yl)but-2-enamide;
93) (S,E)-N-(5-(3-(1-((5-chlorothiazol-2-yl)amino)-1-oxopropan-2-yl)phenyl)pyrazin-2-yl)-4-(dimethylamino)but-2-enamide;
94) (E)-4-(2-oxa-5-azabicyclo[2.2.1]heptan-5-yl)-N-(5-(3-((S)-1-((5-chlorothiazol-2-yl)amino)-1-oxopropan-2-yl)phenyl)pyrazin-2-yl)but-2-enamide;
95) (S,E)-4-(4-methylpiperazin-1-yl)-N-(5-(3-(1-oxo-1-((5-(trifluoromethyl)thiazol-2-yl)amino)propan-2-yl)phenyl)pyrazin-2-yl)but-2-enamide;
96) (E)-4-(2-oxa-5-azabicyclo[2.2.1]heptan-5-yl)-N-(5-(3-((S)-1-oxo-1-((5-(trifluoromethyl)thiazol-2-yl)amino)propan-2-yl)phenyl)pyrazin-2-yl)but-2-enamide;
97) (S,E)-N-(5-(3-(1-((5-chlorothiazol-2-yl)amino)-1-oxopropan-2-yl)phenyl)pyrazin-2-yl)-4-(4-methylpiperazin-1-yl)but-2-enamide;
98) (S,E)-N-(5-(3-(l-((5-chlorothiazol-2-yl)amino)-1-oxopropan-2-yl)phenyl)pyrazin-2-yl)-4-morpholinobut-2-enamide;
99) (S,E)-4-(dimethylamino)-N-(3-fluoro-5-(3-(1-oxo-1-((5-(trifluoromethyl)thiazol-2-yl)amino)propan-2-yl)phenyl)pyridin-2-yl)but-2-enamide;
100) (S,E)-4-(dimethylamino)-N-(3-methoxy-5-(3-(1-oxo-1-((5-(trifluoromethyl)thiazol-2-yl)amino)propan-2-yl)phenyl)pyridin-2-yl)but-2-enamide;
101) (S,E)-N-(3-cyano-5-(3-(1-oxo-1-((5-(trifluoromethyl)thiazol-2-yl)amino)propan-2-yl)phenyl)pyridin-2-yl)-4-(dimethylamino)but-2-enamide;
102) (S,E)-N-(5-(3-(1-oxo-1-((5-(trifluoromethyl)thiazol-2-yl)amino)propan-2-yl)phenyl)pyrazin-2-yl)-4-(pyrrolidin-1-yl)but-2-enamide;
103) (S,E)-4-(azetidin-1-yl)-N-(5-(3-(1-oxo-1-((5-(trifluoromethyl)thiazol-2-yl)amino)propan-2-yl)phenyl)pyrazin-2-yl)but-2-enamide;
104) (S,E)-N-(5-(3-(1-((5-cyclobutylthiazol-2-yl)amino)-1-oxopropan-2-yl)phenyl)pyrazin-2-yl)-4-(dimethylamino)but-2-enamide;
105) (S,E)-4-(dimethylamino)-N-(5-(3-(1-((5-(2-hydroxypropan-2-yl)thiazol-2-yl)amino)-1-oxopropan-2-yl)phenyl)pyrazin-2-yl)but-2-enamide;
106) (S,E)-4-(dimethylamino)-N-(5-(3-(1-((5-(dimethylamino)thiazol-2-yl)amino)-1-oxopropan-2-yl)phenyl)pyrazin-2-yl)but-2-enamide;
107) (E)-4-(dimethylamino)-N-(5'-(1-oxo-1-((5-(trifluoromethyl)thiazol-2-yl)amino)propan-2-yl)-[3,3'-bipyridin]-6-yl)but-2-enamide;
108) (E)-N-(5-cyano-5'-(1-oxo-1-((5-(trifluoromethyl)thiazol-2-yl)amino)propan-2-yl)-[3,3'-bipyridin]-6-yl)-4-(dimethylamino)but-2-enamide;
109) (S,E)-4-(dimethylamino)-N-(3-methoxy-5-(3-(1-oxo-1-((5-(trifluoromethyl)thiazol-2-yl)amino)propan-2-yl)phenyl)pyrazin-2-yl)but-2-enamide;
110) (S,E)-2-(3-(1-(4-(dimethylamino)but-2-enoyl)-2,3-dihydro-1H-pyrrolo[2,3-b]pyridin-5-yl)phenyl)-N-(5-(trifluoromethyl)thiazol-2-yl)propanamide;
111) (S,E)-4-(dimethylamino)-N-(5-(3-(1-oxo-1-((5-(trifluoromethyl)thiazol-2-yl)amino)propan-2-yl)phenyl)-3-(trifluoromethyl)pyridin-2-yl)but-2-enamide;
112) (S,E)-4-(dimethylamino)-N-(3-methyl-5-(3-(1-oxo-1-((5-(trifluoromethyl)thiazol-2-yl)amino)propan-2-yl)phenyl)pyridin-2-yl)but-2-enamide;
113) (S,E)-N-(3-chloro-5-(3-(1-oxo-1-((5-(trifluoromethyl)thiazol-2-yl)amino)propan-2-yl)phenyl)pyridin-2-yl)-4-(dimethylamino)but-2-enamide;
114) (S,E)-4-(diethylamino)-N-(5-(3-(1-oxo-1-((5-(trifluoromethyl)thiazol-2-yl)amino)propan-2-yl)phenyl)pyrazin-2-yl)but-2-enamide;
115) (E)-4-((R)-3-fluoropyrrolidin-1-yl)-N-(5-(3-((S)-1-oxo-1-((5-(trifluoromethyl)thiazol-2-yl)amino)propan-2-yl)phenyl)pyrazin-2-yl)but-2-enamide;
116) (S,E)-4-(dimethylamino)-N-(5-(3-(1-oxo-1-((5-(trifluoromethyl)thiazol-2-yl)amino)propan-2-yl)phenyl)pyridin-2-yl)but-2-enamide;
117) (E)-4-((S)-3-fluoropyrrolidin-1-yl)-N-(5-(3-((S)-1-oxo-1-((5-(trifluoromethyl)thiazol-2-yl)amino)propan-2-yl)phenyl)pyrazin-2-yl)but-2-enamide;
118) (E)-4-((S)-2-(methoxymethyl)pyrrolidin-1-yl)-N-(5-(3-((S)-1-oxo-1-((5-(trifluoromethyl)thiazol-2-yl)amino)propan-2-yl)phenyl)pyrazin-2-yl)but-2-enamide;
119) (E)-4-(3-hydroxypyrrolidin-1-yl)-N-(5-(3-((S)-1-oxo-1-((5-(trifluoromethyl)thiazol-2-yl)amino)propan-2-yl)phenyl)pyrazin-2-yl)but-2-enamide;
120) (S,E)-N-(5-(3-(1-oxo-1-((5-(trifluoromethyl)thiazol-2-yl)amino)propan-2-yl)phenyl)pyrazin-2-yl)-4-(piperidin-1-yl)but-2-enamide;
121) (S,E)-N-(3-cyano-5-(3-(1-((5-ethylthiazol-2-yl)amino)-1-oxopropan-2-yl)phenyl)pyridin-2-yl)-4-(dimethylamino)but-2-enamide;
122) (S,E)-2-(3-(1-(4-(dimethylamino)but-2-enoyl)-2,3-dihydro-1H-pyrrolo[2,3-b]pyridin-5-yl)phenyl)-N-(5-ethylthiazol-2-yl)propanamide;
123) (E)-4-(2-oxa-5-azabicyclo[2.2.1]heptan-5-yl)-N-(3-cyano-5-(3-((S)-1-((5-ethylthiazol-2-yl)amino)-1-oxopropan-2-yl)phenyl)pyridin-2-yl)but-2-enamide;
124) (S)-N-(3-cyano-5-(3-(1-oxo-1-((5-(trifluoromethyl)thiazol-2-yl)amino)propan-2-yl)phenyl)pyridin-2-yl)acrylamide;
125) (S,E)-4-(dimethylamino)-N-(5-(3-(1-((5-ethylthiazol-2-yl)amino)-1-oxopropan-2-yl)phenyl)-3-methylpyridin-2-yl)but-2-enamide;
126) (S,E)-2-(3-(1-(4-(dimethylamino)but-2-enoyl)-2-methyl-1H-pyrrolo[2,3-b]pyridin-5-yl)phenyl)-N-(5-ethylthiazol-2-yl)propanamide;
127) (S,E)-2-(3-(1-(4-(dimethylamino)but-2-enoyl)-1H-pyrrolo[2,3-b]pyridin-5-yl)phenyl)-N-(5-ethylthiazol-2-yl)propanamide;
128) (E)-1-(3-(5-cyano-6-(4-(dimethylamino)but-2-enamido)pyridin-3-yl)phenyl)-N-(5-ethylthiazol-2-yl)cyclopropane-1-carboxamide;
129) (S,E)-4-(dimethylamino)-N-(5-(3-(1-((5-ethylthiazol-2-yl)amino)-1-oxopropan-2-yl)phenyl)pyridin-2-yl)-N-methylbut-2-enamide;
130) (S,E)-4-(dimethylamino)-N-(5-(3-(1-((5-ethylthiazol-2-yl)amino)-1-oxopropan-2-yl)phenyl)-3-fluoropyridin-2-yl)but-2-enamide;
131) (E)-4-(dimethylamino)-N-(6-(3-(1-((5-ethylthiazol-2-yl)amino)-1-oxobutan-2-yl)phenyl)pyridin-3-yl)but-2-enamide;
132) (S,E)-N-(3-cyano-5-(3-(1-((5-ethylthiazol-2-yl)amino)-1-oxopropan-2-yl)phenyl)pyridin-2-yl)-4-(dimethylamino)-N-methylbut-2-enamide;
133) (E)-1-(3-(1-(4-(dimethylamino)but-2-enoyl)-2,3-dihydro-1H-pyrrolo[2,3-b]pyridin-5-yl)phenyl)-N-(5-ethylthiazol-2-yl)cyclopropane-1-carboxamide;
134) (E)-1-(3-(6-(4-(dimethylamino)but-2-enamido)-5-fluoropyridin-3-yl)phenyl)-N-(5-ethylthiazol-2-yl)cyclopropane-1-carboxamide;
135) (S,E)-N-(3-cyano-5-(3-(1-((5-ethylthiazol-2-yl)amino)-1-oxopropan-2-yl)phenyl)pyridin-2-yl)-4-morpholinobut-2-enamide;
136) (S,E)-N-(3-cyano-5-(3-(l-((5-ethylthiazol-2-yl)amino)-1-oxopropan-2-yl)phenyl)pyridin-2-yl)-4-(4-methylpiperazin-1-yl)but-2-enamide;
137) (E)-N-(5-cyano-5'-(1-((5-ethylthiazol-2-yl)amino)-1-oxopropan-2-yl)-[3,3'-bipyridin]-6-yl)-4-(dimethylamino)but-2-enamide;
138) (E)-N-(3-cyano-5-(3-(1-((5-ethylthiazol-2-yl)amino)-1-oxobutan-2-yl)phenyl)pyridin-2-yl)-4-(dimethylamino)but-2-enamide;
139) (S)-2-(3-(5-cyano-6-(2-cyanoacetamido)pyridin-3-yl)phenyl)-N-(5-ethylthiazol-2-yl)propanamide;
140) N-(5-(6-(1-((5-methylthiazol-2-yl)amino)-1-oxopropan-2-yl)pyrazin-2-yl)pyridin-2-yl)acrylamide;
141) N-(2'-(1-((5-methylthiazol-2-yl)amino)-1-oxopropan-2-yl)-[3,4'-bipyridin]-6-yl)acrylamide;
142) N-(4-(3-(1-((5-methylthiazol-2-yl)amino)-1-oxopropan-2-yl)piperidin-1-yl)phenyl)acrylamide;
143) N-(5-(2-(1-((5-methylthiazol-2-yl)amino)-1-oxopropan-2-yl)thiazol-4-yl)pyridin-2-yl)acrylamide;
144) (S)-N-(3-cyclopropyl-5-(3-(1-((5-ethylthiazol-2-yl)amino)-1-oxopropan-2-yl)phenyl)pyridin-2-yl)acrylamide;
145) (S)-N-(3-((2-(dimethylamino)ethyl)(methyl)amino)-3'-(1-((5-ethylthiazol-2-yl)amino)-1-oxopropan-2-yl)-[1,1'-biphenyl]-4-yl)acrylamide;
146) (S)-N-(5-ethylthiazol-2-yl)-2-(3-(6-(vinylsulfonamido)pyridin-3-yl)phenyl)propanamide;
147) (S)-N-(3-(3-(1-((5-ethylthiazol-2-yl)amino)-1-oxopropan-2-yl)phenyl)-1-methyl-1H-pyrazol-5-yl)acrylamide;
148) (S)-N-(4-(3-(1-((5-ethylthiazol-2-yl)amino)-1-oxopropan-2-yl)phenyl)-1H-imidazol-2-yl)acrylamide; and
149) (S)-N-(4-(3-(1-((5-ethylthiazol-2-yl)amino)-1-oxopropan-2-yl)phenyl)thiazol-2-yl)acrylamide.

16. A pharmaceutical composition for preventing or treating cancer, comprising a compound of Formula 2 below or a pharmaceutically acceptable salt thereof: 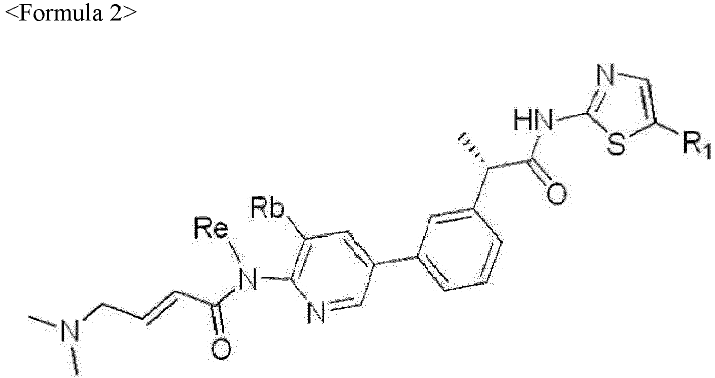 in the formula,
R1 is a linear C₁-C₃ alkyl unsubstituted or substituted with halogen; and
Rb is cyano or halo, and Re is H; or
Rb and Re are fused together with the pyridine linked to Rb and the nitrogen linked to Re to form 2,3-dihydro-1H-pyrrolo[2,3-b]pyridine.

17. The pharmaceutical composition for preventing or treating cancer according to claim 16, **characterized in that** the compound of Formula 2 is selected from the group consisting of the following compounds:
(S,E)-4-(dimethylamino)-N-(3-fluoro-5-(3-(1-oxo-1-((5-(trifluoromethyl)thiazol-2-yl)amino)propan-2-yl)phenyl)pyridin-2-yl)but-2-enamide;
(S,E)-N-(3-cyano-5-(3-(1-oxo-1-((5-(trifluoromethyl)thiazol-2-yl)amino)propan-2-yl)phenyl)pyridin-2-yl)-4-(dimethylamino)but-2-enamide;
(S,E)-2-(3-(1-(4-(dimethylamino)but-2-enoyl)-2,3-dihydro-1H-pyrrolo[2,3-b]pyridin-5-yl)phenyl)-N-(5-(trifluoromethyl)thiazol-2-yl)propanamide;
(S,E)-N-(3-chloro-5-(3-(1-oxo-1-((5-(trifluoromethyl)thiazol-2-yl)amino)propan-2-yl)phenyl)pyridin-2-yl)-4-(dimethylamino)but-2-enamide;
(S,E)-N-(3-cyano-5-(3-(1-((5-ethylthiazol-2-yl)amino)-1-oxopropan-2-yl)phenyl)pyridin-2-yl)-4-(dimethylamino)but-2-enamide;
(S,E)-2-(3-(1-(4-(dimethylamino)but-2-enoyl)-2,3-dihydro-1H-pyrrolo[2,3-b]pyridin-5-yl)phenyl)-N-(5-ethylthiazol-2-yl)propanamide; and
(S,E)-4-(dimethylamino)-N-(5-(3-(1-((5-ethylthiazol-2-yl)amino)-1-oxopropan-2-yl)phenyl)-3-fluoropyridin-2-yl)but-2-enamide.

18. The pharmaceutical composition for preventing or treating cancer according to claim 1 or 16, **characterized in that** the cancer is at least one selected from the group consisting of acoustic neuroma, adenocarcinoma, adrenal cancer, anal cancer, angiosarcoma, appendix cancer, benign monoclonal gammopathy, biliary tract cancer, bladder cancer, breast cancer, brain cancer, bronchial cancer, carcinoid tumor, cervical cancer, choriocarcinoma, chordoma, craniopharyngioma, colorectal cancer, connective tissue cancer, epithelial carcinoma, ependymoma, endothelial sarcoma, endometrial cancer, esophageal cancer, Ewing's sarcoma, eye cancer, familial hypereosinophilia, gallbladder cancer, gastric cancer, gastrointestinal stromal tumor (GIST), germ cell cancer, head and neck cancer, oral cancer, throat cancer, hematopoietic cancer, acute myeloid leukemia (AML), acute promyelocytic leukemia, acute lymphocytic leukemia, acute B cell lymphocytic leukemia, acute T cell lymphocytic leukemia, lymphoma, Burkitt lymphoma, immunoblastic large cell lymphoma, chronic myeloid leukemia (CML), multiple myeloma (MM), heavy chain disease, hemangioblastoma, hypopharyngeal cancer, inflammatory myofibroblastic tumor, immune cell amyloidosis, kidney cancer, liver cancer, lung cancer, leiomyosarcoma (LMS), mastocytosis, muscle cancer, myelodysplastic syndrome (MDS), mesothelioma, myeloproliferative disorder (MPD), neuroblastoma, neurofibroma, neuroendocrine cancer, osteosarcoma, ovarian cancer, papillary adenocarcinoma, pancreatic cancer, penile cancer, pineal tumor, primitive neuroectodermal tumor (PNT), plasma cell neoplasm, paraneoplastic syndrome, intraepithelial neoplasm, prostate cancer, rectal cancer, rhabdomyosarcoma, salivary gland cancer, skin cancer, small intestine cancer, soft tissue sarcoma, sebaceous gland carcinoma, large intestine cancer, sweat gland carcinoma, synovioma, testicular cancer, thyroid cancer, urethral cancer, vaginal cancer, and vulvar cancer.

19. The pharmaceutical composition for preventing or treating cancer according to claim 1 or 16, **characterized in that** the cancer is at least one selected from the group consisting of breast cancer, liver cancer, ovarian cancer, pancreatic cancer, prostate cancer, lung cancer, large intestine cancer, cervical cancer, hematopoietic cancer, acute myeloid leukemia (AML), acute promyelocytic leukemia, acute lymphocytic leukemia, acute B cell lymphocytic leukemia, acute T cell lymphocytic leukemia, lymphoma, Burkitt lymphoma, immunoblastic large cell lymphoma, chronic myeloid leukemia (CML), and multiple myeloma.

20. The pharmaceutical composition for preventing or treating cancer according to claim 1 or 16, **characterized in that** the cancer is at least one selected from the group consisting of breast cancer, hematopoietic cancer, liver cancer, and lung cancer.
